(19) Europäisches Patentamt
European Patent Office
Office européen des brevets

(11) Veröffentlichungsnummer: **0 359 077**
**A1**

# (12) EUROPÄISCHE PATENTANMELDUNG

(21) Anmeldenummer: **89116253.9**

(22) Anmeldetag: **02.09.89**

(51) Int. Cl.5: **C07D 213/30 , C07D 239/26 , A01N 43/40 , A01N 43/54**

(30) Priorität: **15.09.88 DE 3831402**

(43) Veröffentlichungstag der Anmeldung:
**21.03.90 Patentblatt 90/12**

(84) Benannte Vertragsstaaten:
**BE CH DE FR GB IT LI NL SE**

(71) Anmelder: **BAYER AG**

**D-5090 Leverkusen 1 Bayerwerk(DE)**

(72) Erfinder: **Elbe, Hans-Ludwig, Dr.**
**Dasnöckel 59**
**D-5600 Wuppertal 11(DE)**
Erfinder: **Lantzsch, Reinhard, Dr.**
**Am Buschhäuschen 51**
**D-5600 Wuppertal 1(DE)**
Erfinder: **Dutzmann, Stefan, Dr.**
**Leinenweberweg 33**
**D-4000 Düsseldorf 13(DE)**

## (54) Substituierte Pyrimidyl- bzw. Pyridylalkinole, Verfahren zu ihrer Herstellung und ihre Verwendung in Schädlingsbekämpfungsmitteln.

(57) Substituierte Pyrimidyl- bzw. Pyridylalkinole der allgemeinen Formel (I),

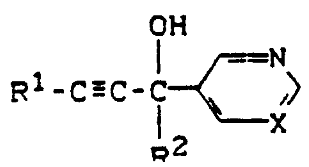

(I)

in welcher
$R^1$, $R^2$ und X die in der Beschreibung gegebenen Bedeutungen haben, ihre Säureadditionssalze und Metallsalzkomplexe und ihre Verwendung zur Bekämpfung von Schädlingen, vor allem pflanzenpathogenen Pilzen. Die neuen substituierten Pyrimidyl- bzw. Pyridylalkinole sind durch die Formel (I) allgemein definiert und sie können z.B. aus geeigneten Pyri(mi)dylketonen mit geeigneten Acetylenverbindungen oder aus geeigneten Acetylenketonen mit geeigneten metallorganischen Heterocyclen hergestellt werden.

EP 0 359 077 A1

## Substituierte Pyrimidyl- bzw. Pyridylalkinole, Verfahren zu ihrer Herstellung und ihre Verwendung in Schädlingsbekämpfungsmitteln

Die Erfindung betrifft neue substituierte Pyrimidyl-bzw. Pyridylalkinole, mehrere Verfahren zu ihrer Herstellung und ihre Verwendung in Schädlingsbekämpfungsmitteln.

Es ist bekannt, daß bestimmte substituierte Pyrimidine und Pyridine, wie beispielsweise die Verbindung 2-(4-Chlorphenoxy)-2-methyl-1-(3-pyridyl)-propan-1-ol fungizide Eigenschaften besitzen (vgl. z.B. EP 221 844).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Weiterhin ist bekannt, daß die Verbindung 1-Cyclohexyl-3-phenyl-1-(3-pyridyl)-propin-1-ol fungizid wirksam ist.

Die Wirksamheit dieser vorbekannten Verbindungen ist jedoch insbesondere bei niedrigen Aufwandmengen und Konzentrationen nicht in allen Anwendungsgebieten völlig zufriedenstellend, wobei auch insbesondere die Breite des Wirkungsspektrums nicht zufriedenstellt (vgl. z.B. Pestic. Sci. 13, 670-678 [1982]).

Es wurden neue substituierte Pyrimidyl- bzw. Pyridylalkinole der allgemeinen Formel (I),

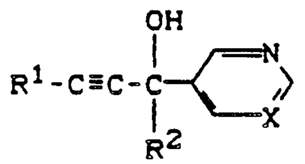

(I)

in welcher

$R^1$ für Alkyl, Alkoxyalkyl, Alkylthioalkyl oder für jeweils unsubstituiertes oder jeweils substituiertes Aryl, Aryloxyalkyl oder Arylthioalkyl steht,

$R^2$ für unsubstituiertes oder substituiertes Cycloalkyl, für Dioxolanyl oder Dioxanyl steht oder für einen der Reste

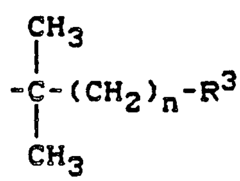 oder 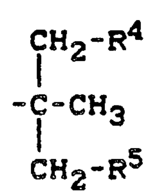 steht und

X für Stickstoff oder eine CH-Gruppe steht,

wobei

$R^3$ für Alkoxy, Alkylthio, Halogenalkoxy, Halogenalkylthio, für Alkenyl, Alkoxycarbonyl oder Cyano, für Dioxolanyl oder Dioxanyl oder für jeweils unsubstituiertes oder jeweils substituiertes Aryl, Aryloxy, Arylthio, Arylalkyloxy oder Arylalkylthio steht,

$R^4$ für Wasserstoff oder Halogen steht,

$R^5$ für Wasserstoff oder Halogen steht und

n für eine Zahl 0, 1 oder 2 steht,

ausgenommen die Verbindung 1-Cyclohexyl-3-phenyl-1-(3-pyridyl-propin-1-ol,

sowie deren Säureadditionssalze und Metallsalzkomplexe gefunden.

Die Verbindung der Formel (I) können als optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Pyrimidyl- bzw. Pyridylalkinole der allgemeinen Formel (I),

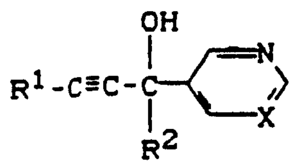 (I)

in welcher

$R^1$ für Alkyl, Alkoxyalkyl, Alkylthioalkyl oder für jeweils unsubstituiertes oder jeweils substituiertes Aryl, Aryloxyalkyl oder Arylthioalkyl steht,

$R^2$ für unsubstituiertes oder substituiertes Cycloalkyl, für Dioxolanyl oder Dioxanyl steht oder für einen der Reste

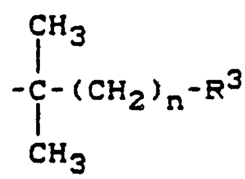 oder 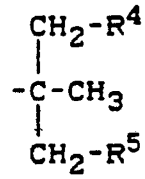 steht und

X für Stickstoff oder eine CH-Gruppe steht,

wobei

$R^3$ für Alkoxy, Alkylthio, Halogenalkoxy, Halogenalkylthio, für Alkenyl, Alkoxycarbonyl oder Cyano, für Dioxolanyl oder Dioxanyl oder für jeweils unsubstituiertes oder jeweils substituiertes Aryl, Aryloxy, Arylthio, Arylalkyloxy oder Arylalkylthio steht,

$R^4$ für Wasserstoff oder Halogen steht,

$R^5$ für Wasserstoff oder Halogen steht und

n für eine Zahl 0, 1 oder 2 steht,

ausgenommen die Verbindung 1-Cyclohexyl-3-phenyl-1-(3-pyridyl)-propin-1-ol,

sowie deren Säureadditionssalze und Metallsalzkomplexe erhält, wenn man

(a) Pyri(mi)dylketone der Formel (II),

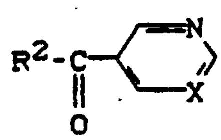 (II)

in welcher

$R^2$ und X die oben angegebene Bedeutung haben,

mit Acetylenverbindungen der Formel (III),

$R^1\text{-}C\equiv C\text{-}H$ (III)

in welcher

$R^1$ die oben angegebene Bedeutung hat,

gegebenenfalls in Gegenwart eines Verdünnungsmittels und gegebenenfalls in Gegenwart eines Reaktionshilfsmittels umsetzt oder wenn man

(b) Acetylenketone der Formel (IV),

$R^1\text{-}C\equiv C\text{-}\underset{\underset{O}{\|}}{C}\text{-}R^2$ (IV)

in welcher

$R^1$ und $R^2$ die oben angegebene Bedeutung haben,

mit metallorganischen Heterocyclen der Formel (V),

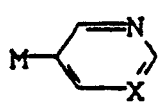 (V)

in welcher
M für Lithium oder für einen Rest -Mg-Hal steht, wobei Hal für Halogen steht und
X die oben angegebene Bedeutung hat,
gegebenenfalls in Gegenwart eines Verdünnungsmittels umsetzt und gegebenenfalls anschließend eine Säure oder ein Metallsalz addiert.

Schließlich wurde gefunden, daß die neuen substituierten Pyrimidyl- oder Pyridylalkinole der allgemeinen Formel (I) sowie deren Säureadditionssalze und Metallsalzkomplexe eine gute Wirksamkeit gegen Schädlinge besitzen.

Überraschenderweise zeigen die erfindungsgemäßen substituierten Pyrimidyl- bzw. Pyridylalkinole der allgemeinen Formel (I) eine erheblich bessere Wirksamkeit gegen Schädlinge, insbesondere gegen pflanzenschädigende Pilze als die aus dem Stand der Technik bekannten substituierten Pyrimidine und Pyridine, wie beispielsweise die Verbindung 2-(4-Chlorphenoxy)-2-methyl-1-(3-pyridyl)-propan-1-ol, welche chemisch und wirkungsmäßig naheliegende Verbindungen sind.

In den allgemeinen Formeln bedeutet Alkyl im allgemeinen geradkettiges oder verzweigtes Alkyl mit 1 bis 6 Kohlenstoffatomen, bevorzugt mit 1 bis 4 Kohlenstoffatomen, beispielhaft seien genannt Methyl, Ethyl, n- und i-Propyl, n-, i-, s- und t-Butyl, Pentyl und Hexyl.

Alkoxyalkyl und Alkylthioalkyl stehen im allgemeinen für einen durch ein Sauerstoff- bzw. Schwefelatom unterbrochenen, geradkettigen oder verzweigten Kohlenwasser stoffrest mit 1 bis 6 Kohlenstoffatomen, in jedem Alk.rest, bevorzugt mit 1 bis 4, besonders bevorzugt mit 1 oder 2 Kohlenstoffatomen je Alk.rest, beispielhaft seien genannt Methoxymethyl, Ethoxymethyl, n- und i-Propoxymethyl, n-, s-, t- und i-Butyloxymethyl, Pentoxymethyl, Methoxyethyl, Ethoxyethyl, n- und i-Propoxyethyl, n-, s-, t- und i-Butoxyethyl, Methoxypropyl, Ethoxypropyl, n- und i-Propoxypropyl, Butoxypropyl, Methoxy-n-butyl, Ethoxy-n-butyl, n- und i-Propoxy-n-butyl, Methylthiomethyl, Ethylthiomethyl, n- und i-Propylthiomethyl, Butylthiomethyl, Methylthioethyl, Ethylthioethyl, n- und i-Propylthioethyl, Butylthioethyl, Methylthiopropyl, Ethylthiopropyl, n- und i-Propylthiopropyl.

Alkoxy steht im allgemeinen für einen über Sauerstoff gebundenen, geradkettigen oder verzweigten Kohlenwasserstoffrest mit 1 bis 6 Kohlenstoffatomen, bevorzugt mit 1 bis 4, besonders bevorzugt mit 1 oder 2 Kohlenstoffatomen; beispielhaft seien genannt Methoxy, Ethoxy, n- Propoxy, i-Propoxy, n-Butoxy, i-Butoxy, s-Butoxy, t-Butoxy, n-Pentoxy, i-Pentoxy, n-Hexoxy und i-Hexoxy.

Alkylthio steht im allgemeinen für einen über Schwefel gebundenen, geradkettigen oder verzweigten Kohlenwasserstoffrest mit 1 bis 6 Kohlenstoffatomen, bevorzugt mit 1 bis 4, besonders bevorzugt mit 1 oder 2 Kohlenstoffatomen; beispielhaft seien genannt Methylthio, Ethylthio, n-Propylthio, i-Propylthio, n-Butylthio, i-Butylthio, s-Butylthio, t-Butylthio, Pentylthio und Hexylthio.

Halogenalkoxy und Halogenalkylthio stehen im allgemeinen für einen über Sauerstoff bzw. Schwefel gebundenen, geradkettigen oder verzweigten Halogenkohlenwasserstoffrest mit 1 bis 6 Kohlenstoffatomen und 1 bis 12 gleichen oder verschiedenen Halogenatomen, bevorzugt mit 1 bis 4 Kohlenwasserstoffatomen und 1 bis 9 gleichen oder verschiedenen Halogenatomen, besonders bevorzugt mit 1 oder 2 Kohlenstoffatomen und 1 bis 3 gleichen oder verschiedenen Halogenatomen, wobei als Halogenatome Fluor, Chlor, Brom und Jod, bevorzugt Fluor, Chlor und Brom, besonders bevorzugt Fluor und Chlor genannt seien; beispielhaft seien genannt Fluormethoxy, Chlormethoxy, Brommethoxy, Fluorethoxy, Chlorethoxy, Bromethoxy, Fluorpropoxy, Chlorpropoxy, Brompropoxy, Fluorbutoxy, Chlorbutoxy, Fluor-i-propoxy, Chlor-i-propoxy, Difluormethoxy, Trifluormethoxy, Dichlormethoxy, Trichlormethoxy, Difluorethoxy, Trifluorethoxy, Tetrafluorethoxy, Trichlorethoxy, Chlordifluormethoxy, Trifluorchlorethoxy und Fluormethylthio, Chlormethylthio, Brommethylthio, Fluorethylthio, Chlorethylthio, Bromethylthio, Fluorpropylthio, Chlorpropylthio, Brompropylthio, Fluorbutylthio, Chlorbutylthio, Fluor-isopropylthio, Chlor-isopropylthio, Chlorbutylthio, Difluormethylthio, Trifluormethylthio, Dichlormethylthio, Trichlormethylthio, Difluorethylthio, Trifluorethylthio, Tetrafluorethylthio, Trichlorethylthio, Chlordifluormethylthio und Trifluorchlorethylthio.

Alkenyl steht im allgemeinen für einen geradkettigen oder verzweigten Kohlenwasserstoffrest mit 2 bis 6 Kohlenstoffatomen und einer oder mehreren Doppelbindungen, bevorzugt ist ein Alkenylrest mit 2 bis 5 Kohlenstoffatomen und 1 Doppelbindung, besonders bevorzugt mit 2 oder 3 Kohlenstoffatomen und 1 Doppelbindung; beispielhaft seien genannt Vinyl, Allyl, n-Propenyl, i-Propenyl, Butenyl, i-Butenyl, n-Pentenyl, i-Pentenyl, n-Hexenyl und Isohexenyl.

Aryl steht im allgemeinen für einen aromatischen Rest mit 6 bis 10 Kohlenstoffatomen. Arylreste sind Phenyl oder Naphthyl, bevorzugt Phenyl.

Aryloxy und Arylthio stehen im allgemeinen für einen aromatischen, über Sauerstoff bzw. Schwefel gebundenen Rest mit 6 bis 10 Kohlenstoffatomen. Arylreste sind Phenyl oder Naphthyl, bevorzugt Phenyl.

Aryloxyalkyl und Arylthioalkyl stehen im allgemeinen für einen aromatischen, über Sauerstoff bzw. Schwefel an Alkyl gebundenen Rest mit 6 bis 10 Kohlenstoffatomen im Arylteil und 1 bis 6 Kohlenstoffatomen im geradkettigen oder verzweigten Alkylteil, bevorzugt für Aryl mit 6 bis 10 Kohlenstoffatomen und 1 bis 4 Kohlenstoffatomen im Alkylteil, ganz besonders bevorzugt mit 6 Kohlenstoffatomen im Arylteil und 1 oder 2 Kohlenstoffatomen im Alkylteil. Arylreste sind Phenyl oder Naphthyl, bevorzugt Phenyl. Beispielsweise seien genannt Phenyloxymethyl, Naphthyloxymethyl, Phenyloxyethyl, Naphthyloxyethyl, Phenyloxy-n-propyl, Phenyloxy-n-butyl, Phenylthiomethyl, Naphthylthiomethyl, Phenylthioethyl, Naphthylthioethyl, Phenylthio-n-propyl, Phenylthio-n-butyl und Phenylthio-i-butyl.

Arylalkyloxy und Arylalkylthio stehen im allgemeinen für einen Aralkylrest mit 6 bis 10 Kohlenstoffatomen im Arylteil und 1 bis 6 Kohlenstoffatomen im geradkettigen oder verzweigten Alkylteil über Sauerstoff bzw. Schwefel gebunden, bevorzugt mit 1 bis 4 und besonders bevorzugt mit 1 Kohlenstoffatom im Alkylteil. Arylalkylreste sind Phenylalkyl und Naphthylalkyl, bevorzugt Phenylalkyl; beispielhaft seien genannt Benzyloxy, Naphthylmethyloxy, Phenylethyloxy, Phenylpropyloxy, Benzylthio, Phenethylthio und Phenylpropylthio.

Cycloalkyl steht im allgemeinen für mono-, bi- und tricyclisches Cycloalkyl mit 3 bis 10, bevorzugt für monocyclisches mit 3 bis 7, besonders bevorzugt für monocyclisches Cycloalkyl mit 3 bis 6 Kohlenstoffatomen; beispielhaft seien genannt Cyclopropyl, Cyclobutyl, Cyclopentyl, Cyclohexyl, Cycloheptyl, Bicyclo[2.2.1]heptyl, und Bicyclo[2.2.2]octyl.

Alkoxycarbonyl steht im allgemeinen für einen geradkettigen oder verzweigten Alkoxyrest mit 1 bis 7 Kohlenstoffatomen, bevorzugt mit 1 bis 5, besonders bevorzugt mit 1 oder 2 Kohlenstoffatomen im Alkoxyrest, der an eine Ketogruppe gebunden ist. Beispielhaft seien genannt Methoxycarbonyl, Ethoxycarbonyl, Propoxycarbonyl, i-Propoxycarbonyl, Butoxycarbonyl, Pentoxycarbonyl, Hexoxycarbonyl und Heptoxycarbonyl.

Aryl als solches oder in Zusammensetzungen wie Aryloxy, Arylthio, Aryloxyalkyl, Arylthioalkyl, Aralkyloxy und Aralkylthio kann ein- bis mehrfach, bevorzugt ein- bis fünffach, besonders bevorzugt ein- bis dreifach, gleich oder verschieden substituiert sein. Als Substituenten seien beispielhaft genannt:

Halogen, wie Fluor, Chlor, Brom und Jod, bevorzugt und besonders bevorzugt Fluor, Chlor und Brom; Cyano; Nitro; jeweils bevorzugt geradkettiges oder verzweigtes Alkyl, Alkoxy oder Alkylthio mit jeweils 1 bis 4 Kohlenstoffatomen, besonders bevorzugt Alkyl mit 1 bis 4, Alkoxy mit 1 bis 3 und Alkylthio mit 1 oder 2 Kohlenstoffatomen; Halogenalkyl, Halogenalkoxy und Halogenalkylthio bevorzugt mit jeweils 1 bis 4 Kohlenstoffatomen im geradkettigen oder verzweigten Alkylrest und 1 bis 9 gleichen oder verschiedenen Halogenatomen, wie Fluor, Chlor, Brom und Jod, besonders bevorzugt mit 1 oder 2 Kohlenstoffatomen und 1 bis 5 gleichen oder verschiedenen Halogenatomen, wie Fluor oder Chlor; Alkoxycarbonyl, Hydroximinoalkyl oder Alkoximinoalkyl mit jeweils bevorzugt 1 bis 3 Kohlenstoffatomen in den geradkettigen oder verzweigten Alkylteilen, besonders bevorzugt mit 1 oder 2 Kohlenstoffatomen je Alkylteil; Phenyl, Phenoxy, Benzyl oder Benzyloxy; ein- bis mehrfach, bevorzugt ein- bis fünffach, besonders bevorzugt ein- bis dreifach, gleich oder verschieden durch Halogen und/oder geradkettiges oder verzweigtes Alkyl bevorzugt mit 1 bis 4, besonders bevorzugt mit 1 oder 2 Kohlenstoffatomen substituiertes Phenyl, Phenoxy, Benzyl oder Benzyloxy.

Halogen hat die oben angegebene Bedeutung.

Cycloalkyl kann ein- bis mehrfach, bevorzugt ein- bis achtfach, besonders bevorzugt ein- bis fünffach, gleich oder verschieden substituiert sein durch Halogen, wie Fluor, Chlor, Brom und Jod, besonders bevorzugt durch Fluor, Chlor und Brom, und geradkettiges oder verzweigtes Alkyl mit 1 bis 4, besonders bevorzugt mit 1 oder 2 Kohlenstoffatomen.

Die erfindungsgemäßen substituierten Pyrimidyl- bzw. Pyridylalkinole sind durch die Formel (I) allgemein definiert. Bevorzugt sind Verbindungen der Formel (I), bei welchen

$R^1$ für jeweils geradkettiges oder verzweigtes Alkyl, Alkoxyalkyl oder Alkylthioalkyl mit jeweils 1 bis 4 Kohlenstoffatomen in den einzelnen Alkylteilen oder für jeweils unsubstituiertes oder jeweils im Arylteil einfach oder mehrfach, gleich oder verschieden substituiertes Aryl, Aryloxyalkyl oder Arylthioalkyl mit jeweils 6 bis 10 Kohlenstoffatomen im Arylteil und gegebenenfalls 1 bis 4 Kohlenstoffatomen im geradkettigen oder verzweigten Alkylteil steht,

wobei als Substituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl, Hydroximinoalkyl oder Alkoximinoalkyl mit jeweils 1 bis 3 Kohlenstoffatomen in den einzelnen Alkylteilen und jeweils unsubstituiertes oder einfach bis mehrfach, gleich oder verschieden durch Halogen und/oder geradkettiges oder verzweigtes Alkyl mit 1 bis 4 Kohlenstoffatomen substituiertes Phenyl, Phenoxy, Benzyl oder Benzyloxy;

$R^2$ für unsubstituiertes oder einfach bis mehrfach, gleich oder verschieden durch Halogen und/oder

geradkettiges oder verzweigtes Alkyl mit 1 bis 4 Kohlenstoffatomen substituiertes Cycloalkyl mit 3 bis 7 Kohlenstoffatomen, für Dioxolanyl, für Dioxanyl oder für einen Rest der Formel

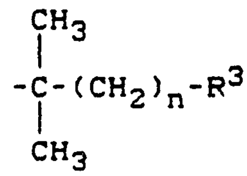

oder für einen Rest der Formel

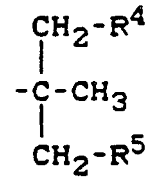 steht und

X für Stickstoff oder eine CH-Gruppe steht,
wobei
$R^3$ für jeweils geradkettiges oder verzweigtes Alkoxy oder Alkylthio mit jeweils 1 bis 4 Kohlenstoffatomen, für jeweils geradkettiges oder verzweigtes Halogenalkoxy oder Halogenalkylthio mit jeweils 1 bis 4 Kohlenstoffatomen und 1 bis 9 gleichen oder verschiedenen Halogenatomen, für geradkettiges oder verzweigtes Alkenyl mit 2 bis 5 Kohlenstoffatomen, für geradkettiges oder verzweigtes Alkoxycarbonyl mit 2 bis 5 Kohlenstoffatomen, für Cyano, für Dioxolanyl, für Dioxanyl oder für jeweils unsubstituiertes oder jeweils im Arylteil einfach bis mehrfach, gleich oder verschieden substituiertes Aryl, Aryloxy, Arylthio, Arylalkyloxy oder Arylalkylthio mit jeweils 6 bis 10 Kohlenstoffatomen im Arylteil und gegebenenfalls 1 bis 4 Kohlenstoffatomen im geradkettigen oder verzweigten Alkylteil steht, wobei als Arylsubstituenten die bei $R^1$ genannten infrage kommen,
$R^4$ für Wasserstoff, Fluor, Chlor, Brom oder Iod steht,
$R^5$ für Wasserstoff, Fluor, Chlor, Brom oder Iod steht und
n für eine Zahl 0, 1 oder 2 steht,
ausgenommen die Verbindung 1-Cyclohexyl-3-phenyl-1-(3-pyridyl)-propin-1-ol.

Besonders bevorzugt sind Verbindungen der Formel (I), bei welchen
$R^1$ für Methyl, Ethyl, n- oder i-Propyl, n-, i-, s-oder t-Butyl, für Methoxymethyl, Ethoxymethyl, Methylthiomethyl, Ethylthiomethyl, für jeweils unsubstituiertes oder jeweils ein- bis dreifach, gleich oder verschieden substituiertes Phenyl, Phenoxymethyl, Phenoxyethyl oder Phenylthiomethyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Trifluormethyl, Trifluormethoxy, Trifluormethylthio, Trifluorethyl, Trichlorethyl, Difluorchlorethyl, Methoxycarbonyl, Ethoxycarbonyl, Hydroximinomethyl, Hydroximinoethyl, Methoximinomethyl, Ethoximinomethyl, Methoximinoethyl, Ethoximinoethyl oder jeweils unsubstituiertes oder jeweils ein- bis dreifach, gleich oder verschieden durch Fluor, Chlor, Brom, Methyl und/oder Ethyl substituiertes Phenyl, Phenoxy, Benzyl oder Benzyloxy;
$R^2$ für jeweils unsubstituiertes oder jeweils ein- bis fünffach, gleich oder verschieden durch Fluor, Chlor, Brom, Methyl und/oder Ethyl substituiertes Cyclopropyl, Cyclobutyl, Cyclopentyl oder Cyclohexyl, für Dioxolanyl, Dioxanyl oder für einen Rest der Formel

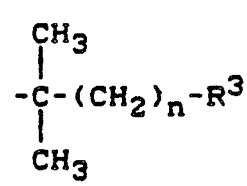

oder für einen Rest der Formel

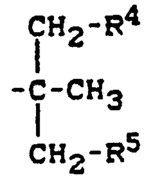

steht und

X für Stickstoff oder eine CH-Gruppe steht,

wobei

$R^3$ für Methoxy, Ethoxy, Methylthio, Ethylthio, Trifluormethoxy, Trifluormethylthio, für Trifluorethoxy, Trifluorethylthio, Trifluorchlorethoxy, für Vinyl, Propenyl, Ethoxycarbonyl, für Cyano, für Dioxolanyl, Dioxanyl oder für jeweils unsubstituiertes oder jeweils einbis dreifach, gleich oder verschieden substituiertes Phenyl, Phenoxy, Phenylthio, Benzyloxy oder Benzylthio steht, wobei als Phenylsubstituenten jeweils die bei $R^1$ genannten infrage kommen,

$R^4$ für Wasserstoff, Fluor, Chlor oder Brom steht,

$R^5$ für Wasserstoff, Fluor, Chlor oder Brom steht und

n für eine Zahl 0, 1 oder 2 steht,

ausgenommen die Verbindung 1-Cyclohexyl-3-phenyl-1-(3-pyridyl)-propin-1-ol.

Bevorzugte erfindungsgemäße Verbindungen sind auch Additionsprodukte aus Säuren und denjenigen substituierten Pyrimidyl- bzw. Pyridylalkinolen der Formel (I), in denen die Substituenten $R^1$, $R^2$ und X die Bedeutungen haben, die bereits vorzugsweise für diese Substituenten genannt wurden.

Zu den Säuren die addiert werden können, gehören vorzugsweise Halogenwasserstoffsäuren, wie z.B. Chlorwasserstoffsäure und Bromwasserstoffsäure, insbesondere Chlorwasserstoffsäure, ferner Phosphorsäure, Salpetersäure, Schwefelsäure, mono-, bi- und trifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, Sulfonsäuren, wie z.B. p-Toluolsulfonsäure und 1,5-Naphthalindisulfonsäure sowie Saccharin oder Thiosaccharin.

Außerdem bevorzugte erfindungsgemäße Verbindungen sind Additionsprodukte aus Salzen von Metallen der II. bis IV. Haupt- und der I. und II. sowie IV. bis VIII. Neben gruppe und denjenigen substituierten Pyrimidyl- bzw. Pyridylalkinolen der Formel (I), in denen die Substituenten $R^1$, $R^2$ und X die Bedeutung haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu pflanzenverträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, Salpetersäure und Schwefelsäure.

Im einzelnen seien außer den bei den Herstellungsbeispielen genannten Verbindungen die folgenden substituierten Pyrimidyl- bzw. Pyridylalkinole der allgemeinen Formel (I) genannt:

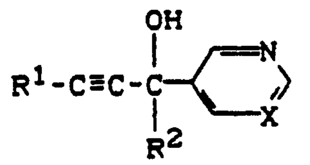

(I)

| $R^1$ | $R^2$ | X |
|---|---|---|
| 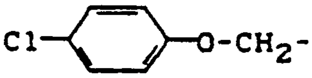 | 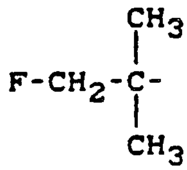 | N |
| 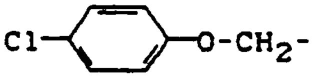 | 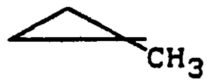 | N |
| 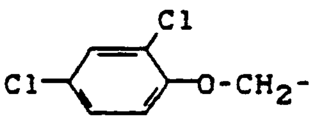 | $(CH_3)_3C$- | N |
| 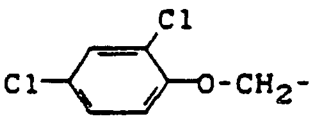 | 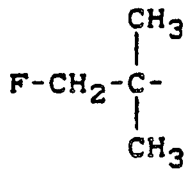 | N |
| 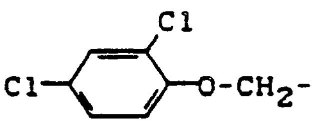 | 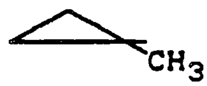 | N |
| 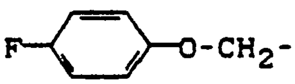 | $(CH_3)_3C$- | N |
| 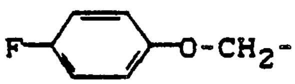 | 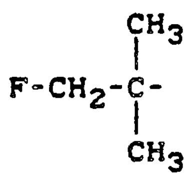 | N |
| 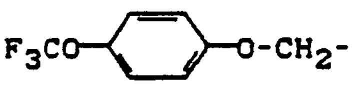 | $(CH_3)_3C$- | N |
| 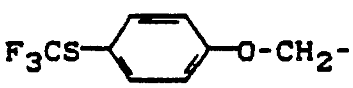 | $(CH_3)_3C$- | N |

| $R^1$ | $R^2$ | X |
|---|---|---|
| 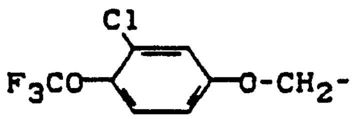 | $(CH_3)_3C-$ | N |
| 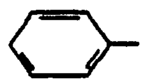 | $(CH_3)_3C-$ | N |
| $CH_3O-CH_2-$ | $(CH_3)_3C-$ | N |
| $CH_3S-CH_2-$ | $(CH_3)_3C-$ | N |
| $CH_3O-CH_2-$ | 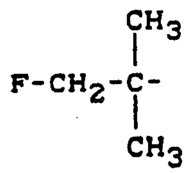 | N |
| $CH_3S-CH_2-$ | 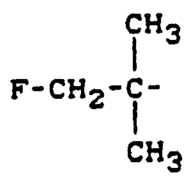 | N |
| 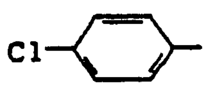 | $(CH_3)_3C-$ | N |
| 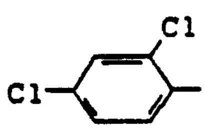 | $(CH_3)_3C-$ | N |
| 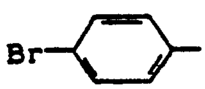 | $(CH_3)_3C-$ | N |
| 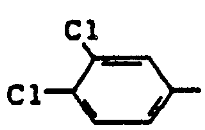 | $(CH_3)_3C-$ | N |

| $R^1$ | $R^2$ | X |
|---|---|---|
| 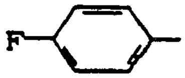 | $(CH_3)_3C-$ | N |
| 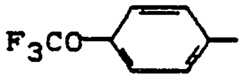 | $(CH_3)_3C-$ | N |
| 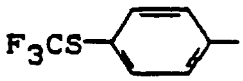 | $(CH_3)_3C-$ | N |
| 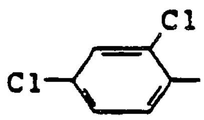 | 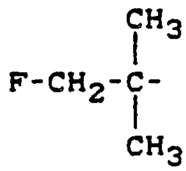 | N |
| 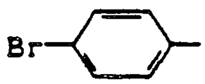 | 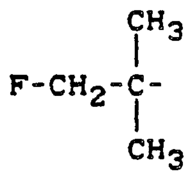 | N |
| 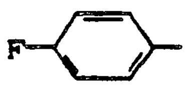 | 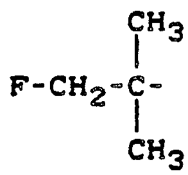 | N |
| 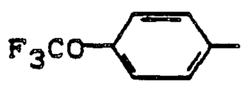 | 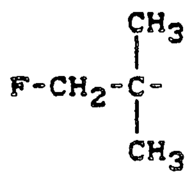 | N |
| 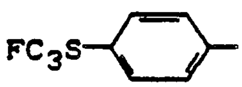 | 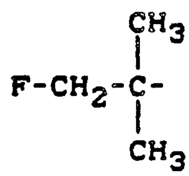 | N |

| $R^1$ | $R^2$ | X |
|---|---|---|
| 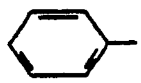 | 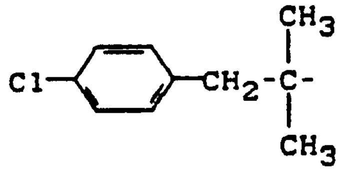 | N |
| 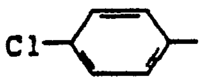 | 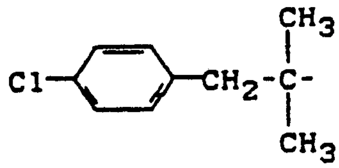 | N |
| 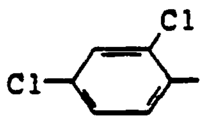 | 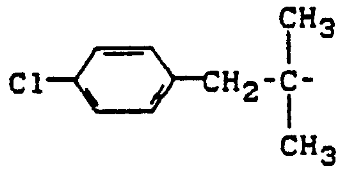 | N |
| 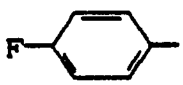 | 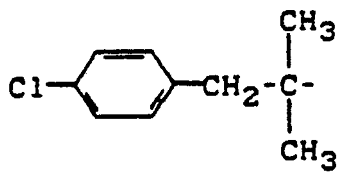 | N |
| $CH_3$ | 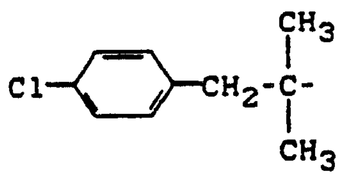 | N |
| $C_2H_5$ | 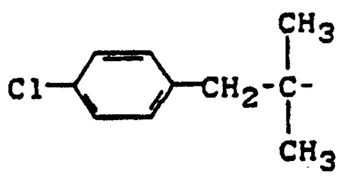 | N |
| 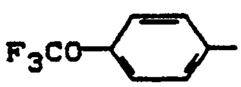 | 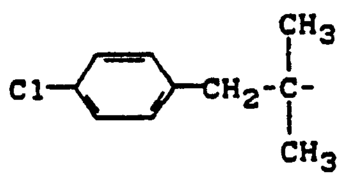 | N |

| $R^1$ | $R^2$ | X |
|---|---|---|
| 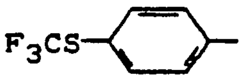 | 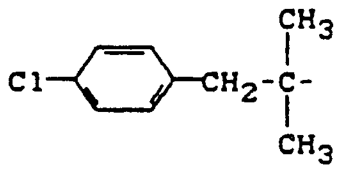 | N |
| 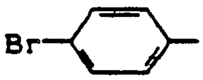 | 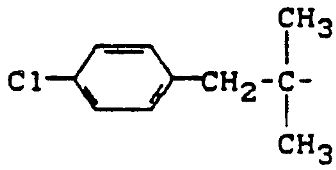 | N |
| 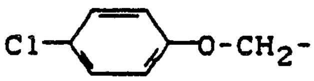 | 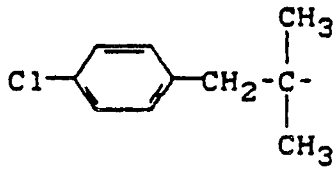 | N |
| $CH_3O-CH_2-$ | 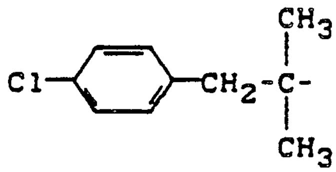 | N |
| $CH_3S-CH_2-$ | 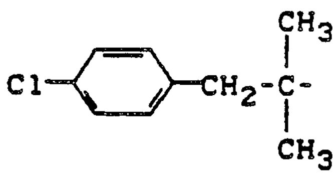 | N |
| 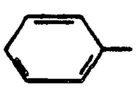 | 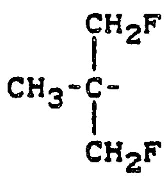 | N |
| 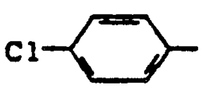 | 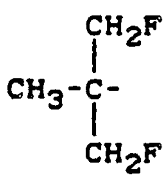 | N |

| $R^1$ | $R^2$ | X |
|---|---|---|
| 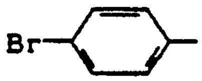 | 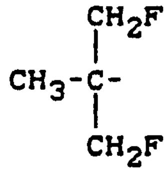 | N |
| $CH_3$ | 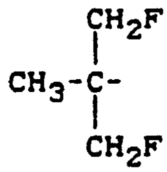 | N |
| $C_2H_5$ | 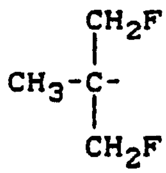 | N |
| $CH_3O-CH_2-$ | 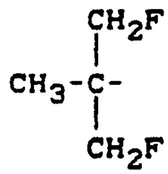 | N |
| $CH_3S-CH_2-$ | 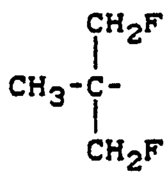 | N |
| 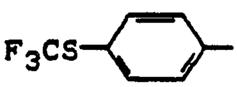 | 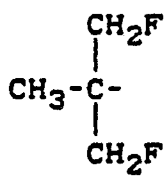 | N |
| 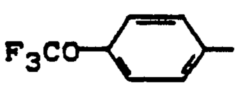 | 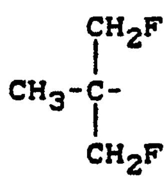 | N |

| $R^1$ | $R^2$ | X |
|---|---|---|
| 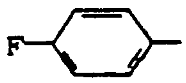 | 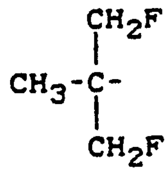 | N |
| 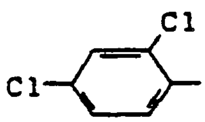 | 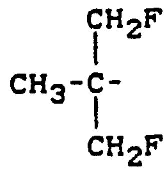 | N |
| 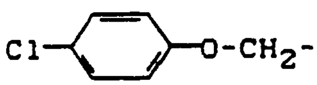 | 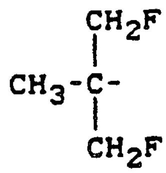 | N |
| 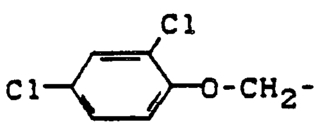 | 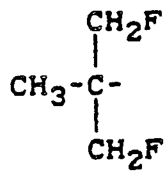 | N |
| 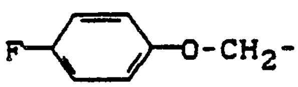 | 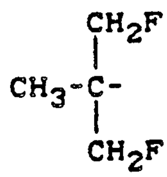 | N |
| 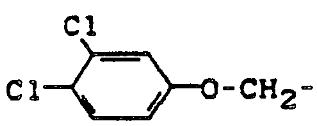 | 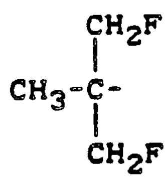 | N |
| $CH_3$ | 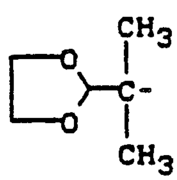 | N |

| $R^1$ | $R^2$ | X |
|---|---|---|
| $C_2H_5$ | 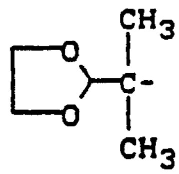 | N |
| $CH_3O\text{-}CH_2\text{-}$ | 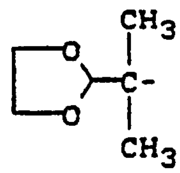 | N |
| 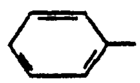 | 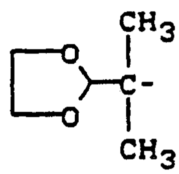 | N |
| 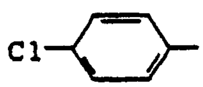 | 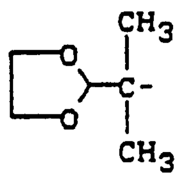 | N |
| 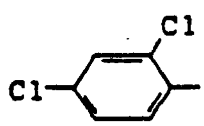 | 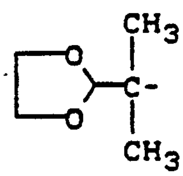 | N |
| 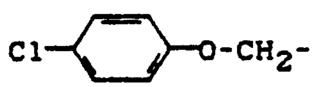 | 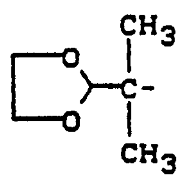 | N |
| $CH_3$ | 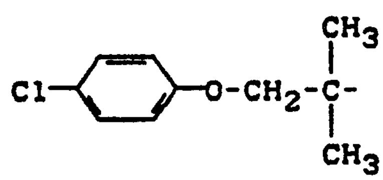 | N |

| $R^1$ | $R^2$ | X |
|---|---|---|
| $C_2H_5$ | 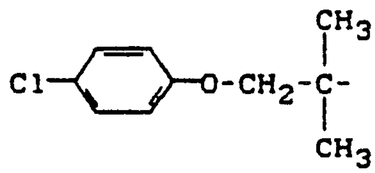 | N |
| $CH_3O-CH_2-$ | 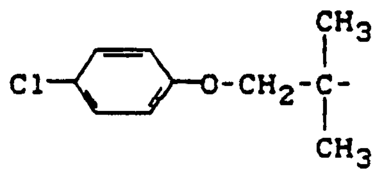 | N |
| $C_2H_5O-CH_2-$ | 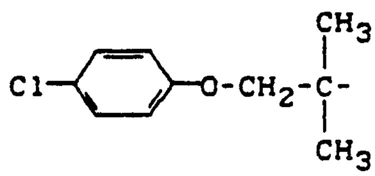 | N |
| 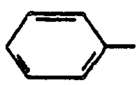 | 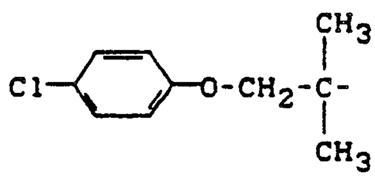 | N |
| 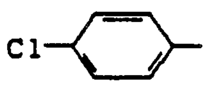 | 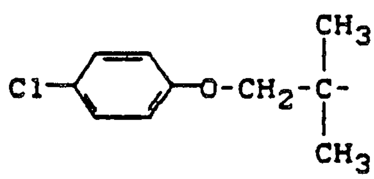 | N |
| 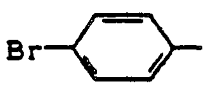 | 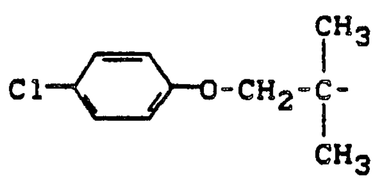 | N |
| 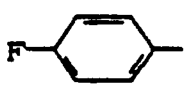 | 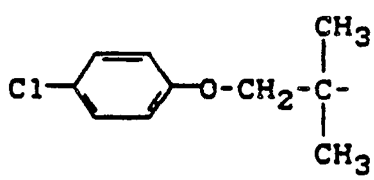 | N |

| $R^1$ | $R^2$ | X |
|---|---|---|
| 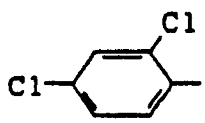 | 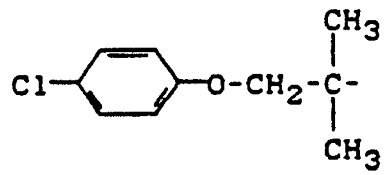 | N |
| 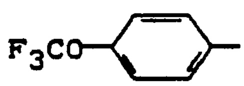 | 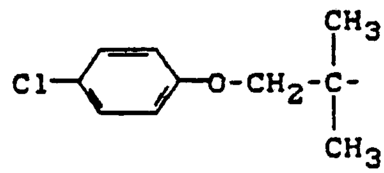 | N |
| 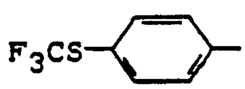 | 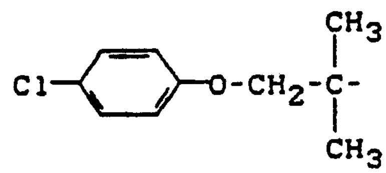 | N |
| 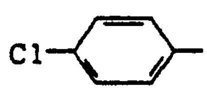 | 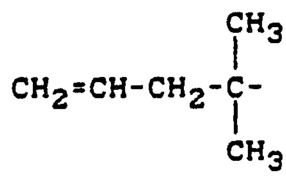 | N |
| 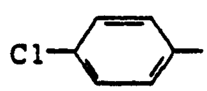 | 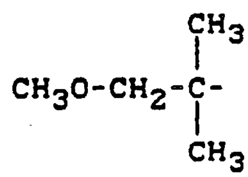 | N |
| 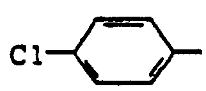 | 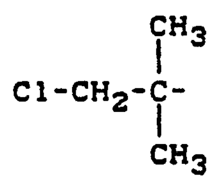 | N |
| 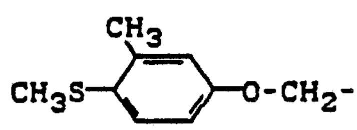 | 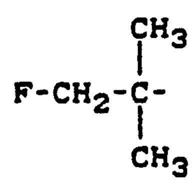 | N |

| $R^1$ | $R^2$ | X |
|---|---|---|
| 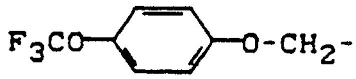 | 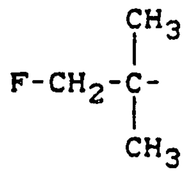 | N |
| 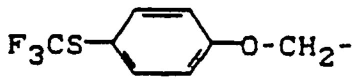 | 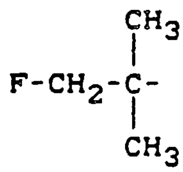 | N |
| 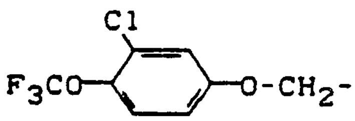 | 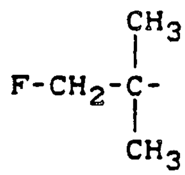 | N |
| 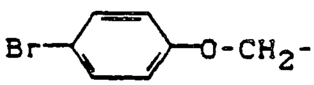 | 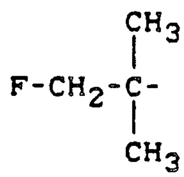 | N |
| 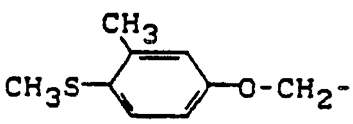 | $(CH_3)_3C-$ | N |
| 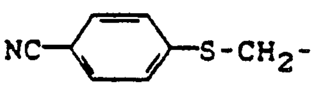 | $(CH_3)_3C-$ | N |
| 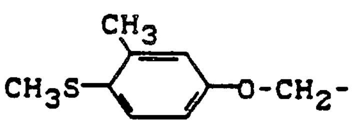 | 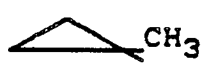 | N |
| 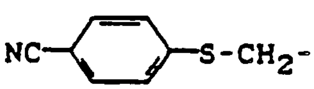 | 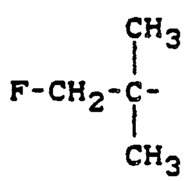 | N |

| $R^1$ | $R^2$ | X |
|---|---|---|
| 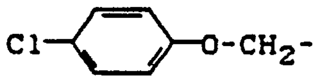 | 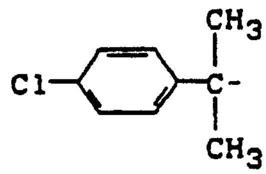 | N |
| 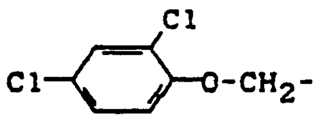 | 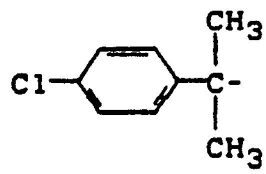 | N |
| 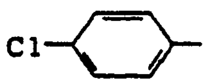 | 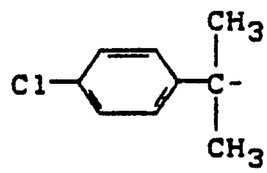 | N |
| 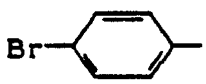 | 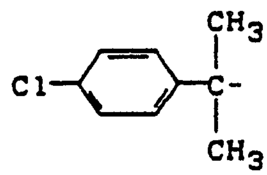 | N |
| 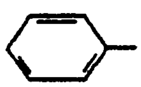 | 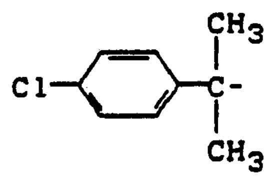 | N |
| $CH_3$ | 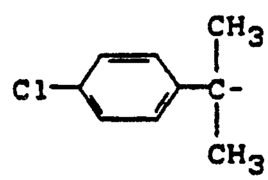 | N |
| $C_2H_5$ | 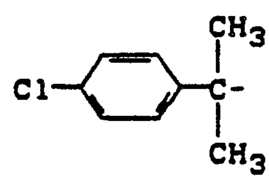 | N |

| $R^1$ | $R^2$ | X |
|---|---|---|
| 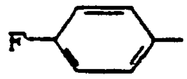 | 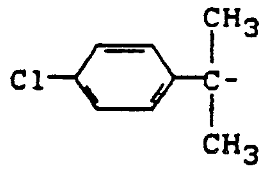 | N |
| 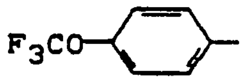 | 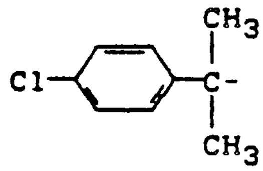 | N |
| 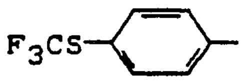 | 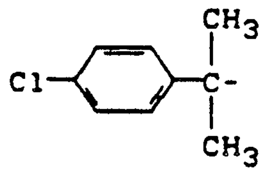 | N |
| 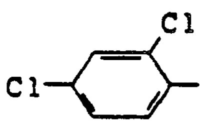 | 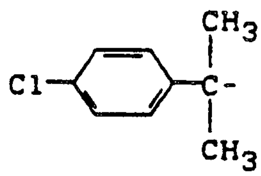 | N |
| 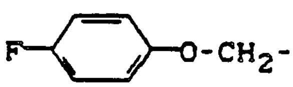 | 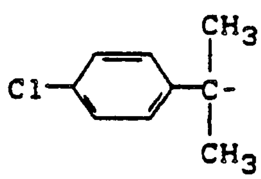 | N |
| 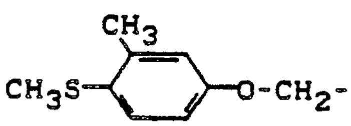 | 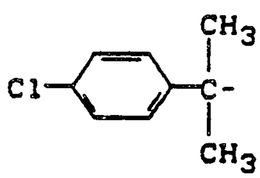 | N |
| 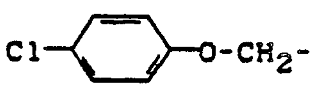 | 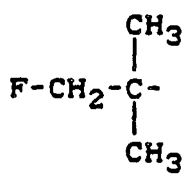 | CH |

| $R^1$ | $R^2$ | X |
| --- | --- | --- |
| 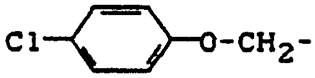 | 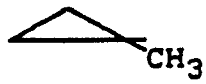 | CH |
| 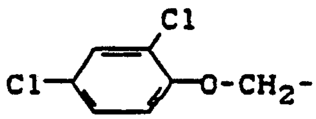 | $(CH_3)_3C-$ | CH |
| 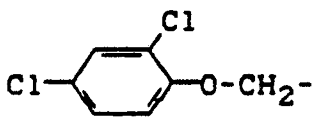 | 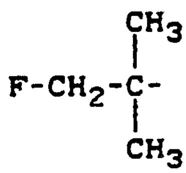 | CH |
| 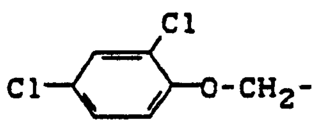 | 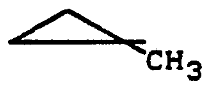 | CH |
| 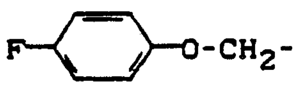 | $(CH_3)_3C-$ | CH |
| 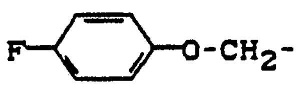 | 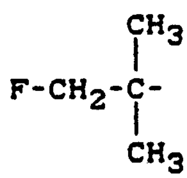 | CH |
| 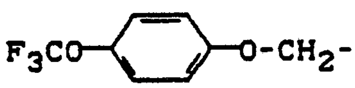 | $(CH_3)_3C-$ | CH |
| 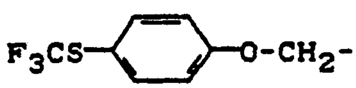 | $(CH_3)_3C-$ | CH |
| 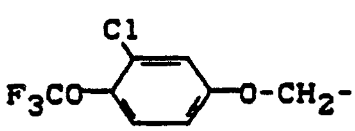 | $(CH_3)_3C-$ | CH |

| $R^1$ | $R^2$ | X |
|---|---|---|
| 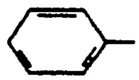 | $(CH_3)_3C-$ | CH |
| $CH_3O-CH_2-$ | $(CH_3)_3C-$ | CH |
| $CH_3S-CH_2-$ | $(CH_3)_3C-$ | CH |
| $CH_3O-CH_2-$ | 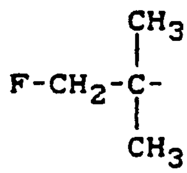 | CH |
| $CH_3S-CH_2-$ | 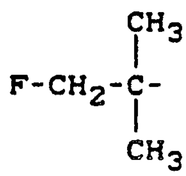 | CH |
| 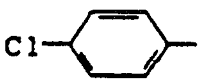 | $(CH_3)_3C-$ | CH |
| 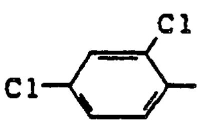 | $(CH_3)_3C-$ | CH |
| 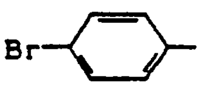 | $(CH_3)_3C-$ | CH |
| 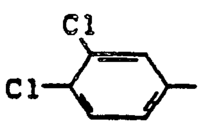 | $(CH_3)_3C-$ | CH |
| 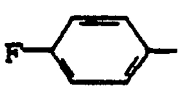 | $(CH_3)_3C-$ | CH |

| $R^1$ | $R^2$ | X |
|---|---|---|
| 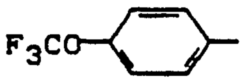 | $(CH_3)_3C-$ | CH |
| 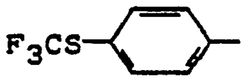 | $(CH_3)_3C-$ | CH |
| 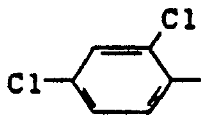 | 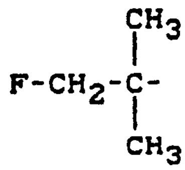 | CH |
| 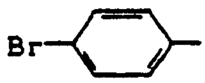 | 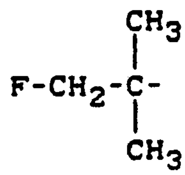 | CH |
| 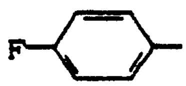 | 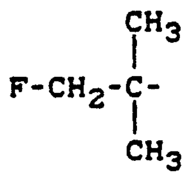 | CH |
| 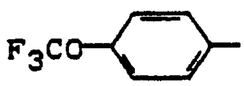 | 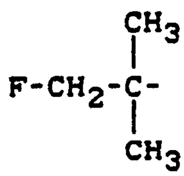 | CH |
| 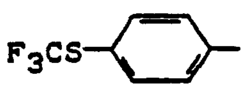 | 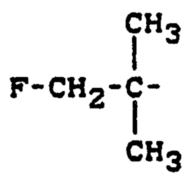 | CH |
| 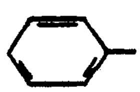 | 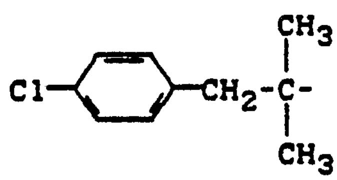 | CH |

| $R^1$ | $R^2$ | X |
| --- | --- | --- |
| 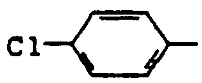 | 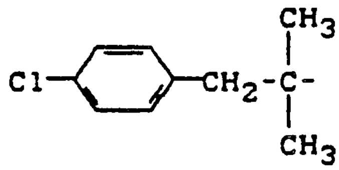 | CH |
| 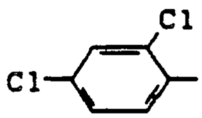 | 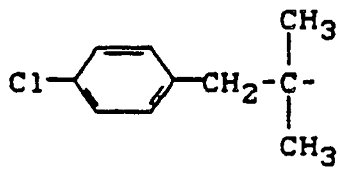 | CH |
| 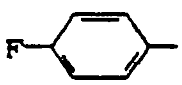 | 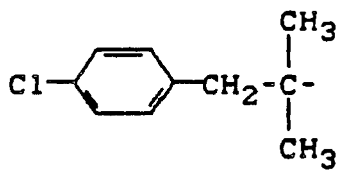 | CH |
| $CH_3$ | 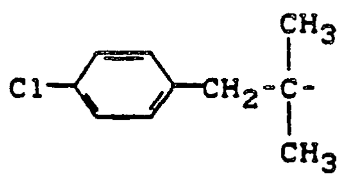 | CH |
| $C_2H_5$ | 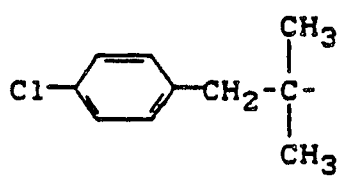 | CH |
| 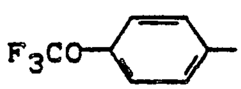 | 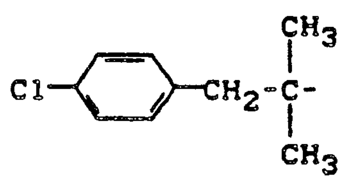 | CH |
| 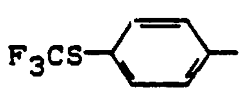 | 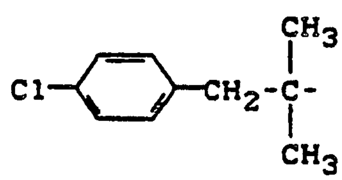 | CH |

| $R^1$ | $R^2$ | X |
|---|---|---|
| 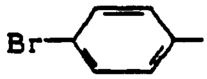 | 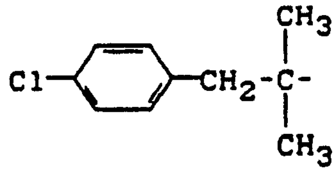 | CH |
| 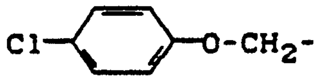 | 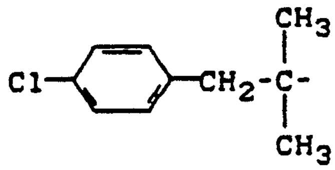 | CH |
| $CH_3O-CH_2-$ | 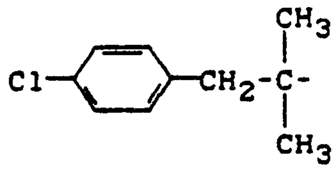 | CH |
| $CH_3S-CH_2-$ | 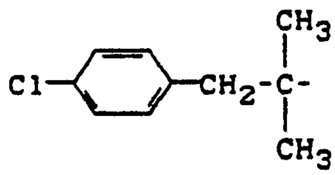 | CH |
| 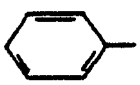 | 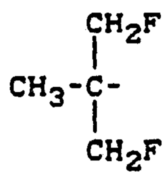 | CH |
| 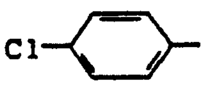 | 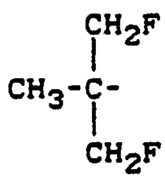 | CH |
| 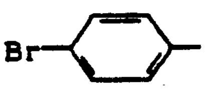 | 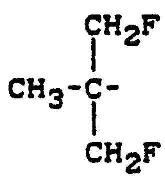 | CH |

| $R^1$ | $R^2$ | X |
|---|---|---|
| $CH_3$ | 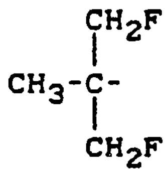 | CH |
| $C_2H_5$ | 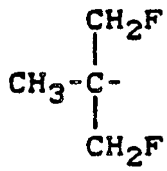 | CH |
| $CH_3O-CH_2-$ | 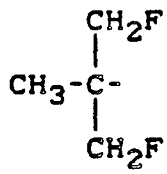 | CH |
| $CH_3S-CH_2-$ | 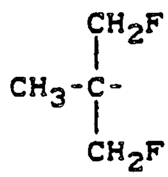 | CH |
| 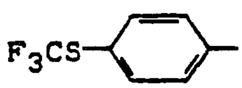 | 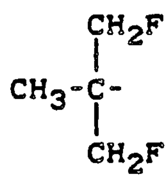 | CH |
| 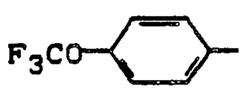 | 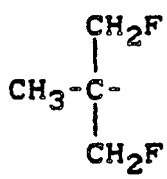 | CH |
| 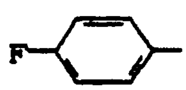 | 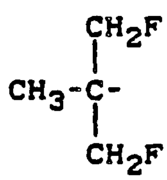 | CH |

| $R^1$ | $R^2$ | X |
| --- | --- | --- |
| 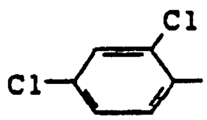 | 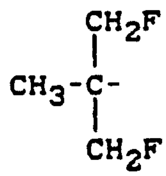 | CH |
| 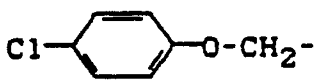 | 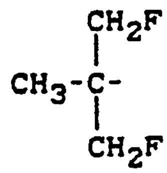 | CH |
| 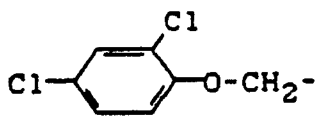 | 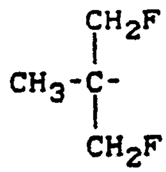 | CH |
| 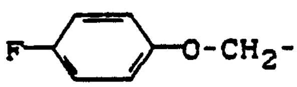 | 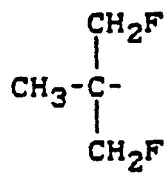 | CH |
| 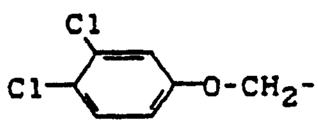 | 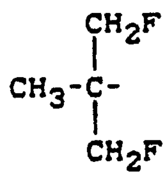 | CH |
| $CH_3$ | 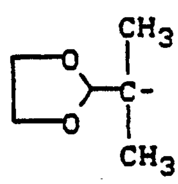 | CH |
| $C_2H_5$ | 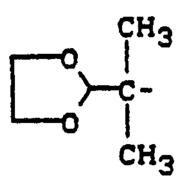 | CH |

| $R^1$ | $R^2$ | X |
|---|---|---|
| $CH_3O-CH_2-$ | 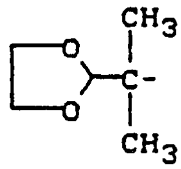 | CH |
| 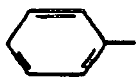 | 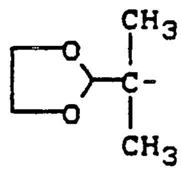 | CH |
| 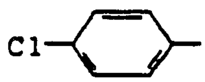 | 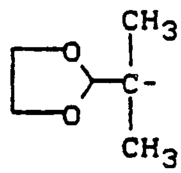 | CH |
| 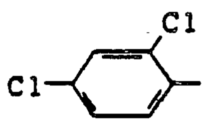 | 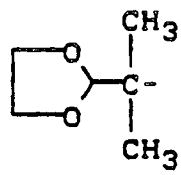 | CH |
| 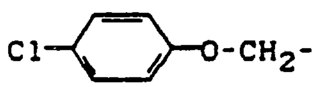 | 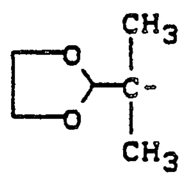 | CH |
| $CH_3$ | 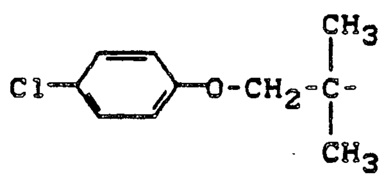 | CH |
| $C_2H_5$ | 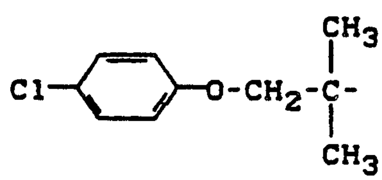 | CH |

| $R^1$ | $R^2$ | X |
|---|---|---|
| $CH_3O-CH_2-$ | 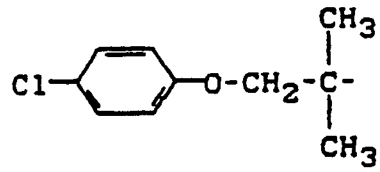 | CH |
| $C_2H_5O-CH_2-$ | 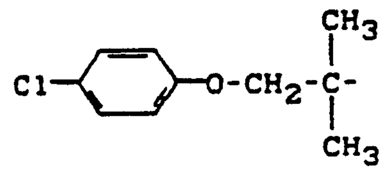 | CH |
| 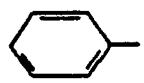 | 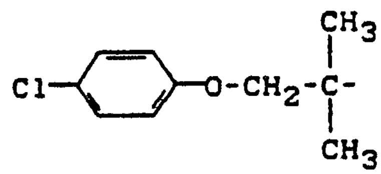 | CH |
| 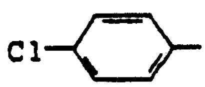 | 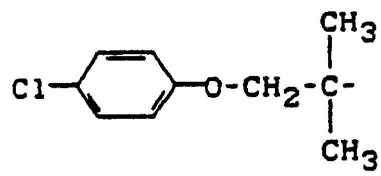 | CH |
| 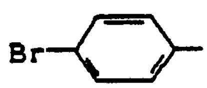 | 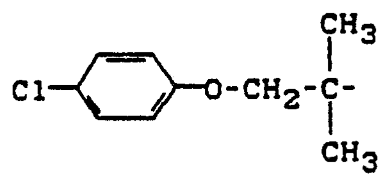 | CH |
| 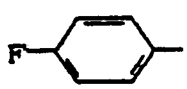 | 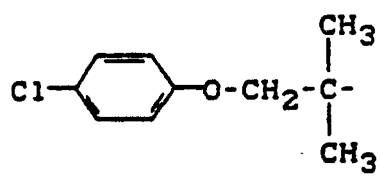 | CH |
| 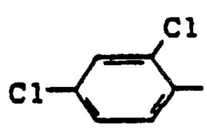 | 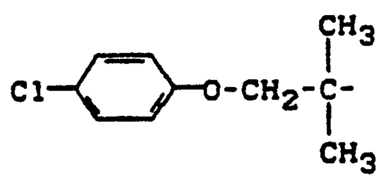 | CH |

| $R^1$ | $R^2$ | X |
| --- | --- | --- |
| 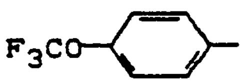 | 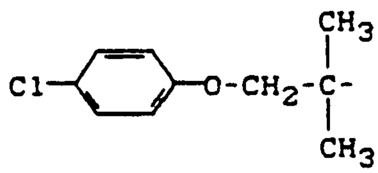 | CH |
| 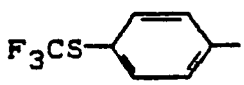 | 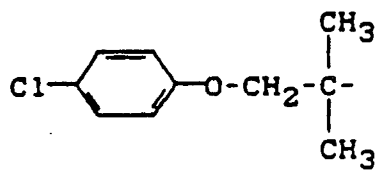 | CH |
| 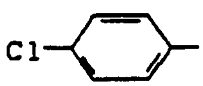 | 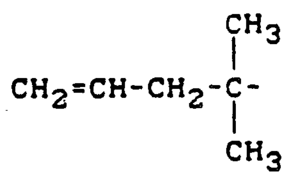 | CH |
| 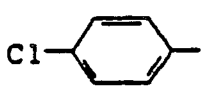 | 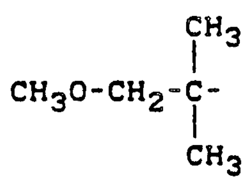 | CH |
| 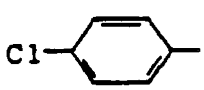 | 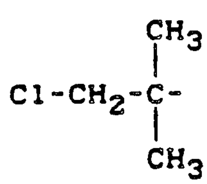 | CH |
| 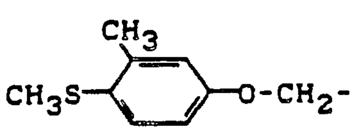 | 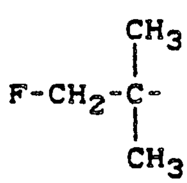 | CH |
| 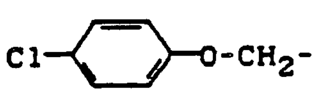 | 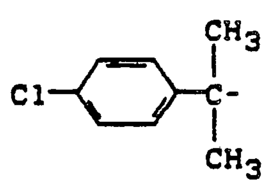 | CH |

| $R^1$ | $R^2$ | X |
|---|---|---|
| 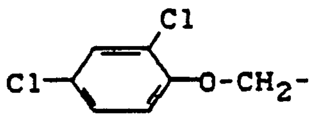 | 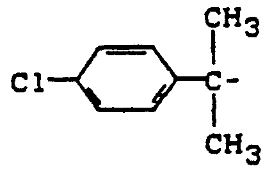 | CH |
| 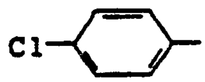 | 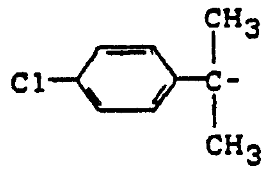 | CH |
| 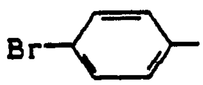 | 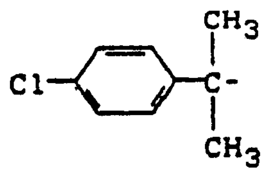 | CH |
| 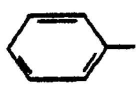 | 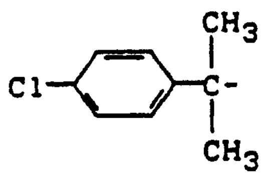 | CH |
| $CH_3$ | 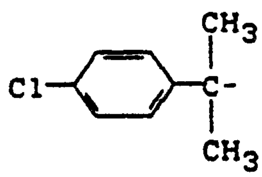 | CH |
| $C_2H_5$ | 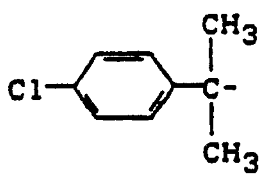 | CH |
| 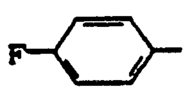 | 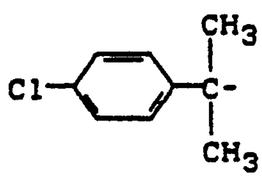 | CH |

| $R^1$ | $R^2$ | X |
|---|---|---|
| 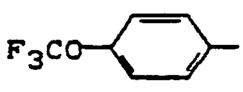 | 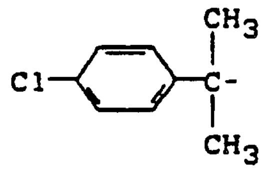 | CH |
| 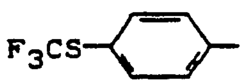 | 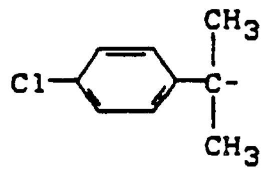 | CH |
| 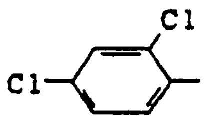 | 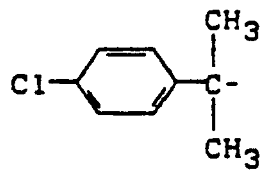 | CH |
| 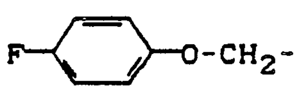 | 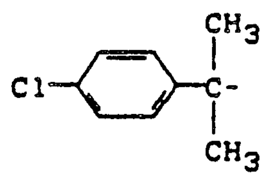 | CH |
| 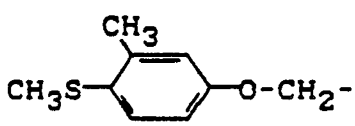 | 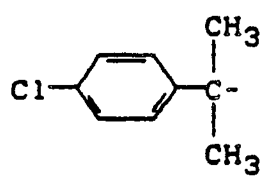 | CH |

Verwendet man beispielsweise 2,2-Dimethyl-1-(5-pyrimidyl)-propan-1-on und Phenylacetylen als Ausgangsstoffe, so läßt sich der Reaktionsablauf des erfindungsgemäßen Verfahrens (a) durch das folgende Formelschema darstellen:

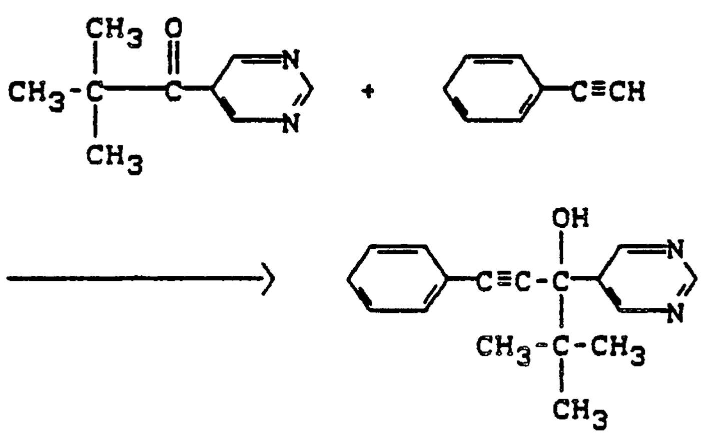

Verwendet man beispielsweise 4,4-Dimethyl-5-fluor-1-phenyl-1-pentin-3-on und 5-Pyrimidyllithium als Ausgangsstoffe, so läßt sich der Reaktionsablauf des erfindungsgemäßen Verfahrens (b) durch das folgende Formelschema darstellen:

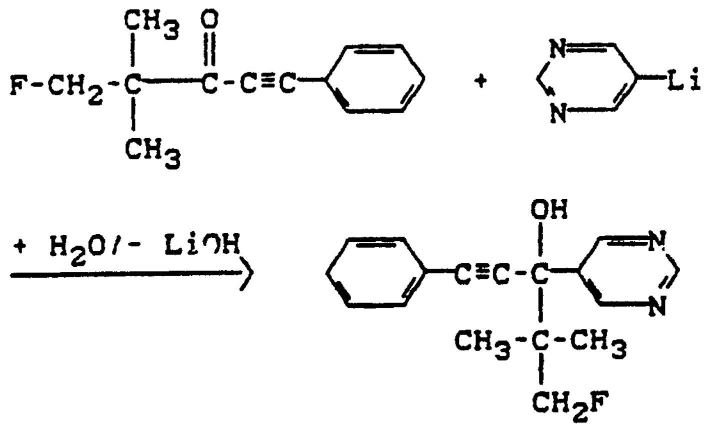


Die zur Durchführung des erfindungsgemäßen Verfahrens (a) als Ausgangsstoffe benötigten Pyri(mi)-dylketone sind durch die Formel (II) allgemein definiert. In dieser Formel (II) stehen $R^2$ und X vorzugsweise für diejenigen Reste, die bereits im Zusammenhang mit der Beschreibung der erfindungsgemäßen Stoffe der Formel (I) als bevorzugt für diese Substituenten genannt wurden.

Die Pyri(mi)dylketone der Formel (II) sind bekannt oder erhältlich in Analogie zu bekannten Verfahren (vgl. z.B. DE-OS 3 431 689; US-PS 4 098 908; DE-OS 2 616 481; Can. J. Chem. 60, 1821-1827 [1982]).

Die zur Durchführung des erfindungsgemäßen Verfahrens (a) weiterhin als Ausgangsstoffe benötigten Acetylenverbindungen sind durch die Formel (III) allgemein definiert. In dieser Formel (III) steht $R^1$ vorzugsweise für diejenigen Reste, die bereits im Zusammenhang mit der Beschreibung der erfindungsgemäßen Stoffe der Formel (I) als bevorzugt für diesen Substituenten genannt wurden.

Die Acetylenverbindungen der Formel (III) sind allgemein bekannte Verbindungen der organischen Chemie (vgl. z.B. Angew. Chem. Int. Ed. Engl. 9, 464 [1970]; Compt. Rend. Acad. Sci. Ser. C; 278, 287-290 [1974]).

Die zur Durchführung des erfindungsgemäßen Verfahrens (b) als Ausgangsstoffe benötigten Acetylenketone sind durch die Formel (IV) allgemein definiert. In dieser Formel (IV) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die Acetylenketone der Formel (IV) sind bekannt oder erhältlich in Analogie zu bekannten Verfahren (vgl. z.B. DE-OS 3 343 531; Tetrahedron Lett. 24, 5131-5132 [1983]; J. org. Chem. 47, 52-56 [1982]; Tetrahedron Lett. 1977, 3151-3154; Synthesis 1977, 777-778; Synth. Commun. 2, 331-334 [1972]; J. Amer. chem. Soc. 93, 2471-2481 [1971]), beispielsweise wenn man Acetylenverbindungen der Formel (III),

$R^1-C\equiv CH$ (III)

in welcher

$R^1$ die oben angegebene Bedeutung hat,

mit Säurehalogeniden der Formel (VI),

$R^2-\overset{O}{\overset{\|}{C}}-Hal^1$ (VI)

in welcher

$Hal^1$ für Halogen, insbesondere für Chlor oder Brom steht und

$R^2$ die oben angegebene Bedeutung hat,

gegebenenfalls in Gegenwart eines Verdünnungsmittels, wie beispielsweise Toluol, und gegebenenfalls in Gegenwart eines Reaktionshilfsmittels, wie beispielsweise Kupfer(I)bromid, sowie gegebenenfalls in Gegenwart eines Säurebindemittels, wie beispielsweise Triethylamin, bei Temperaturen zwischen 0 °C und 150 °C umsetzt (vgl. auch die Herstellungsbeispiele).

Säurehalogenide der Formel (VI) sind allgemein bekannte Verbindungen der organischen Chemie (vgl. z.B. DE-OS 3 611 195 oder DE-OS 3 310 954).

Die zur Durchführung des erfindungsgemäßen Verfahrens (b) weiterhin als Ausgangsstoffe benötigten metallorganischen Verbindungen sind durch die Formel (V) allgemein definiert. In dieser Formel (V) steht X vorzugsweise für diejenigen Reste, die bereits im Zusammenhang mit der Beschreibung der erfindungsgemäßen Stoffe der Formel (I) als bevorzugt für diesen Substituenten genannt wurden.

M steht vorzugsweise für Lithium oder für einen Rest -Mg-Hal, wobei Hal für Halogen, vorzugsweise für Chlor, Brom oder Iod, insbesondere für Brom steht.

Die metallorganischen Verbindungen der Formel (V) sind bekannt (vgl. z.B. EP 221 844; Angew. Chem.

Int. Ed. Engl. 8, 279 [1969]; EP 131 867).

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl- oder -diethylether; Nitrile, wie Acetonitril oder Propionitril; Amide, wie Dimethylformamid, Dimethylacetamid, N-Methylformanilid, N-Methylpyrrolidon oder Hexamethylphosphorsäuretriamid; Sulfoxide, wie Dimethylsulfoxid oder Acetale, wie Butyraldehyddibutylacetal, Acetaldehyddibutylacetal oder 2-Methyl-2-ethyl-1,3-dioxolan.

Das erfindungsgemäße Verfahren (a) wird vorzugsweise in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat oder metallorganische Verbindungen, wie beispielsweise Ethylmagnesiumbromid oder n-Butyllithium

Das erfindungsgemäße Verfahren (a) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyl-dimethyl-ammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid oder Tris-[2-(2-methoxyethoxy)-ethyl]-amin.

Die Reaktionstemperaturen können bei der Durchführung des erfindungsgemäßen Verfahrens (a) in einem größeren Bereich variiert werden. Im allgemeinen arbeitet man bei Temperaturen zwischen - 80 °C und + 150° C vorzugsweise bei Temperaturen zwischen - 10 °C und + 120 °C.

Zur Durchführung des erfindungsgemäßen Verfahrens (a) setzt man pro Mol an Pyri(mi)dylketon der Formel (II) im allgemeinen 1.0 bis 2.0 Mol, vorzugsweise 1.0 bis 1.2 Mol an Acetylenverbindung der Formel (III), gegebenenfalls 1.0 bis 2.0 Mol, vorzugsweise 1.0 bis 1.2 Mol an Reaktionshilfsmittel und gegebenenfalls 0,1 bis 1,0 Mol an Phasentransferkatalysator ein.

Die Reaktionsdurchführung, Aufarbeitung und Isolierung der Reaktionsprodukte erfolgt nach allgemein üblichen Methoden (vgl. z.B. Europ. J. Med. Chem.-Chim. Ther. 9, 651-657 [1974]).

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan oder Cyclohexan; Ether, wie Diethylether, Diisopropylether, Dioxan, Tetrahydrofuran, Ethylenglykoldimethyl- oder -diethylether oder Amide, wie Hexamethylphosphorsäuretriamid.

Die Reaktionstemperaturen können bei der Durchführung des erfindungsgemäßen Verfahrens (b) in einem größeren Bereich variiert werden. Im allgemeinen arbeitet man bei Temperaturen zwischen - 120 °C und + 120 °C, vorzugsweise bei Temperaturen zwischen - 120 °C und + 60 °C.

Zur Durchführung des erfindungsgemäßen Verfahrens (b) setzt man pro Mol an Acetylen-Keton der Formel (IV) im allgemeinen 1.0 bis 3.0 Mol, vorzugsweise 1.0 bis 1.5 Mol an metallorganischer Verbindung der Formel (III) ein. Die Reaktionsdurchführung erfolgt nach allgemein üblichen Methoden.

Dabei kann die Umsetzung erforderlichenfalls in Gegenwart eines geeigneten Inertgases, wie beispielsweise Stickstoff oder Helium durchgeführt werden. Es ist auch möglich, die als Reaktionspartner eingesetzten metallorganischen Verbindungen der Formel (V) aus geeigneten Ausgangsverbindungen, wie beispielsweise 5-Brompyrimidin oder 3-Brompyridin und n-Butyllithium oder Isopropylmagnesiumbromid, in einer vorgelagerten Reaktion direkt im Reaktionsgefäß herzustellen und ohne Isolierung im Eintopfverfahren mit den Acetylen-Ketonen der Formel (IV) weiter umzusetzen.

Die Aufarbeitung und Isolierung der Reaktionsprodukte erfolgt ebenfalls nach üblichen Methoden (vgl. auch die Herstellungsbeispiele).

Die Säureadditionssalze der Verbindungen der Formel (I) können in einfacher Weise nach üblichen Salzbildungsmethoden, wie z.B. durch Lösen einer Verbindung der Formel (I) in einem geeigneten inerten Lösungsmittel und Hinzufügen der Säure, wie z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die Metallsalzkomplexe von Verbindungen der Formel (I) können in einfacher Weise nach üblichen Verfahren erhalten werden, so z.B. durch Lösen des Metallsalzes in Alkohol, z.B. Ethanol und Hinzufügen zur Verbindung der Formel (I). Man kann Metallsalz-Komplexe in bekannter Weise, z.B. durch Abfiltrieren

isolieren und gegebenenfalls durch Umkristallisation reinigen.

Darüberhinaus stellen die erfindungsgemäßen Verbindungen der Formel (I) interessante Zwischenprodukte dar. Sie lassen sich beispielsweise an der Hydroxygruppe in üblicher Art und Weise, wie z.B. durch Veretherung oder Veresterung, derivatisieren oder durch teilweise oder vollständige Hydrierung der Dreifachbindung in weitere interessante Wirkstoffe überführen.

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide, geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturi-Arten, wie beispielsweise Ventura inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger der Braunspelzigkeit des Weizens (Leptosphaeria nodorum) oder gegen den Erreger des echten Getreidemehltaus (Erysiphe graminis) oder gegen den Erreger der Braunfleckenkrankheit an Weizen oder Gerste (Cochliobolus sativus) oder gegen den Erreger der Netzfleckenkrankheit der Gerste (Pyrenophora teres) sowie zur Bekämpfung von Reiskrankheiten, wie beispielsweise gegen den Erreger der Reisfleckenkrankheit (Pyricularia oryzae) oder zur Bekämpfung von Krankheiten im Obst- und Gemüseanbau, wie beispielsweise gegen Gurkenmehltau oder gegen Bohnenrost, einsetzen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Gra nulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als

Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stick stoff und Kohlendioxid; als feste Trägerstoffe kommen Sin Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarb stoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

Herstellungsbeispiele

Beispiel 1

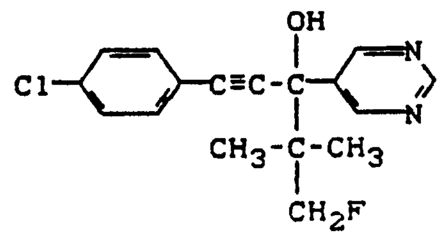

(Verfahren b)

Zu einer Lösung aus 10 g (0,063 Mol) 5-Brompyrimidin in einer Mischung aus 50 ml Diethylether und 50 ml Tetrahydrofuran gibt man bei - 110 °C tropfenweise unter Rühren zunächst eine Lösung von 29,4g (0,069 Mol) n-Butyllithium (15prozentig in n-Hexan) und anschließend bei der selben Temperatur 15 g (0,063 Mol) 1-(4-Chlorphenyl)-5-fluor-4,4-dimethyl-1-pentin-3-on, rührt nach beendeter Zugabe weitere 30 Minuten bei - 110 °C, läßt dann den Ansatz auf Raumtemperatur kommen, gibt tropfenweise 100 ml gesättigte Ammoniumchloridlösung zu, trennt die organische Phase ab, wäscht mit Wasser, trocknet über Natriumsulfat, engt im Vakuum ein und kristallisiert den Rückstand durch Verrühren mit Diisopropylether.

Man erhält 7,1 g (35,4 % der Theorie) an 1-(4-Chlorphenyl)-5-fluor-4,4-dimethyl-3-(5-pyrimidinyl)-1-pentin-3-ol vom Schmelzpunkt 138-140 °C.

Herstellung der Ausgangsverbindung

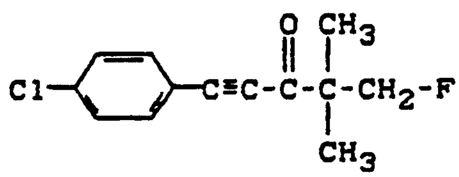

Zu 60,6 g (0,6 Mol) Triethylamin und 8,6 g (0,06 Mol) Kupfer(I)bromid in 300 ml Toluol gibt man bei Raumtemperatur unter einer Stickstoffatmosphäre 81,9 g (0,6 Mol) 4-Chlorphenylacetylen, rührt 30 Minuten bei Raumtemperatur, erwärmt dann auf 50 °C, gibt tropfenweise unter Rühren 83,1 g (0,6 Mol) Monofluorpivalinsäurechlorid zu, erhitzt anschließend auf 90 °C und rührt 10 Stunden bei dieser Temperatur. Zur Aufarbeitung wird auf Raumtemperatur abgekühlt, filtriert, das Filtrat mit verdünnter Salzsäure und Wasser gewaschen, über Natriumsulfat getrocknet, im Vakuum eingeengt und im Hochvakuum destilliert.

Man erhält 118,3 g (80 % der Theorie) an 1-(4-Chlorphenyl)-5-fluor-4,4-dimethyl-1-pentin-3-on vom Siedepunkt 108 bis 112 °C bei 0,1 mbar und vom Schmelzpunkt 64 °C.

In entsprechender Weise und gemäß den allgemeinen Angaben zur Herstellung erhält man die folgenden substituierten Pyrimidyl- bzw. Pyridylalkinole der allgemeinen Formel (I):

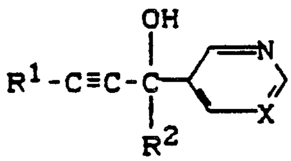 (I)

| Bsp. Nr. | $R^1$ | $R^2$ | X | physikalische Eigenschaften |
|---|---|---|---|---|
| 2 | 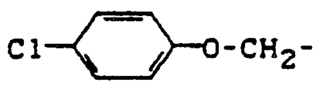 | $(CH_3)_3C$- | CH | $^1$H-NMR*): 4,77 (2H); 0,93 (9H) |
| 3 | 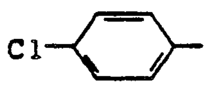 | 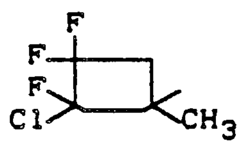 | CH | $n_D^{20}$ 1,5801 |
| 4 | 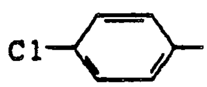 | 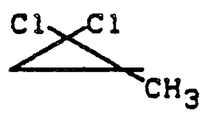 | CH | $n_D^{20}$ 1,6149 |
| 5 | 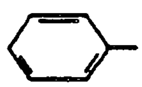 | 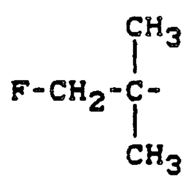 | CH | Fp. 64-67 °C |
| 6 | 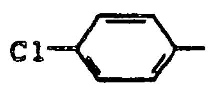 | 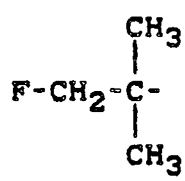 | CH | $n_D^{20}$ 1,5891 |

| Bsp. Nr. | $R^1$ | $R^2$ | X | physikalische Eigenschaften |
|---|---|---|---|---|
| 7 | 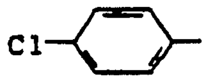 | $(CH_3)_3C-$ | CH | Fp. 184° C |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

## Anwendungsbeispiele

In dem folgenden Anwendungsbeispiel wurde die nachstehend aufgeführte Verbindung als Vergleichsubstanz eingesetzt:

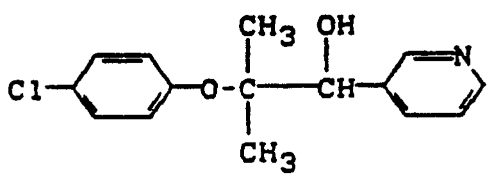

(A)

2-(4-Chlorphenoxy)-2-methyl-1-(3-pyridinyl)-propan-1-ol

(bekannt aus EP 221 844).

### Beispiel A

Leptosphaeria nodorum-Test (Weizen)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit

den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 15 °C und einer relativen Luftfeuchtigkeit von ca. 80 % aufgestellt.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen 6 und 7.

## Ansprüche

1. Substituierte Pyrimidyl- bzw. Pyridylalkinole der allgemeinen Formel (I),

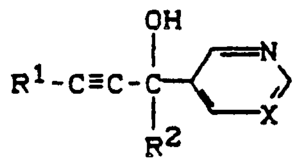

(I)

in welcher

$R^1$ für Alkyl, Alkoxyalkyl, Alkylthioalkyl oder für jeweils unsubstiutiertes oder jeweils substituiertes Aryl, Aryloxyalkyl oder Arylthioalkyl steht,

$R^2$ für unsubstituiertes oder substituiertes Cycloalkyl, für Dioxolanyl oder Dioxanyl steht oder für einen der Reste

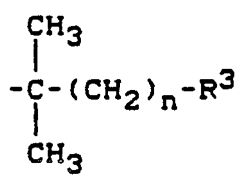

oder

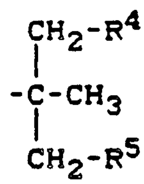

steht und

X für Stickstoff oder eine CH-Gruppe steht,

wobei

$R^3$ für Alkoxy, Alkylthio, Halogenalkoxy, Halogenalkylthio, für Alkenyl, Alkoxycarbonyl oder Cyano, für Dioxolanyl oder Dioxanyl oder für jeweils unsubstituiertes oder jeweils substituiertes Aryl, Aryloxy, Arylthio, Arylalkyloxy oder Arylalkylthio steht,

$R^4$ für Wasserstoff oder Halogen steht,

$R^5$ für Wasserstoff oder Halogen steht und

n für eine Zahl 0, 1 oder 2 steht,

ausgenommen die Verbindung 1-Cyclohexyl-3-phenyl-1-(3-pyridyl)-propin-1-ol,

sowie deren Säureadditionssalze und Metallsalzkomplexe.

2. Substituierte Pyrimidyl- bzw. Pyridylalkinole gemäß Anspruch 1, wobei in der Formel (I)

$R^1$ für jeweils geradkettiges oder verzweigtes Alkyl, Alkoxyalkyl oder Alkylthioalkyl mit jeweils 1 bis 4 Kohlenstoffatomen in den einzelnen Alkylteilen oder für jeweils unsubstituiertes oder jeweils im Arylteil einfach oder mehrfach, gleich oder verschieden substituiertes Aryl, Aryloxyalkyl oder Arylthioalkyl mit jeweils 6 bis 10 Kohlenstoffatomen im Arylteil und gegebenenfalls 1 bis 4 Kohlenstoffatomen im geradkettigen oder verzweigten Alkylteil steht, wobei als Substituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl, Hydroximinoalkyl oder Alkoximinoalkyl mit jeweils 1 bis 3 Kohlenstoffatomen in den einzelnen Alkylteilen und jeweils unsubstituiertes oder einfach bis mehrfach, gleich oder verschieden durch Halogen und/oder geradkettiges oder verzweigtes Alkyl mit 1 bis 4 Kohlenstoffatomen substituiertes Phenyl, Phenoxy, Benzyl oder Benzyloxy;

$R^2$ für unsubstituiertes oder einfach bis mehrfach, gleich oder verschieden durch Halogen und/oder geradkettiges oder verzweigtes Alkyl mit 1 bis 4 Kohlenstoffatomen substituiertes Cycloalkyl mit 3 bis 7 Kohlenstoffatomen, für Dioxolanyl, für Dioxanyl oder für einen Rest der Formel

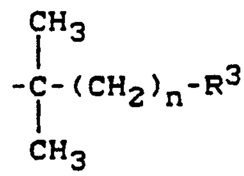

oder für einen Rest der Formel

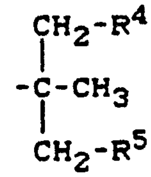

steht und

X für Stickstoff oder eine CH-Gruppe steht,

wobei

$R^3$ für jeweils geradkettiges oder verzweigtes Alkoxy oder Alkylthio mit jeweils 1 bis 4 Kohlenstoffatomen, für jeweils geradkettiges oder verzweigtes Halogenalkoxy oder Halogenalkylthio mit jeweils 1 bis 4 Kohlenstoffatomen und 1 bis 9 gleichen oder verschiedenen Halogenatomen, für geradkettiges oder verzweigtes Alkenyl mit 2 bis 5 Kohlenstoffatomen, für geradkettiges oder verzweigtes Alkoxycarbonyl mit 2 bis 5 Kohlenstoffatomen, für Cyano, für Dioxolanyl, für Dioxanyl oder für jeweils unsubstituiertes oder jeweils im Arylteil einfach bis mehrfach, gleich oder verschieden substituiertes Aryl, Aryloxy, Arylthio, Arylalkyloxy oder Arylalkylthio mit jeweils 6 bis 10 Kohlenstoffatomen im Arylteil und gegebenenfalls 1 bis 4 Kohlenstoffatomen im geradkettigen oder verzweigten Alkylteil steht, wobei als Arylsubstituenten die bei $R^1$ genannten infrage kommen,

$R^4$ für Wasserstoff, Fluor, Chlor, Brom oder Iod steht,

$R^5$ für Wasserstoff, Fluor, Chlor, Brom oder Iod steht und

n für eine Zahl 0, 1 oder 2 steht,

ausgenommen die Verbindung 1-Cyclohexyl-3-phenyl-1-(3-pyridyl)-propin-1-ol.

3. Substituierte Pyrimidyl- bzw. Pyridylalkinole gemäß Anspruch 1, wobei in der Formel (I)

$R^1$ für Methyl, Ethyl, n- oder i-Propyl, n-, i-, s- oder t-Butyl, für Methoxymethyl, Ethoxymethyl, Methylthiomethyl, Ethylthiomethyl, für jeweils unsubstituiertes oder jeweils ein- bis dreifach, gleich oder verschieden substituiertes Phenyl, Phenoxymethyl, Phenoxyethyl oder Phenylthiomethyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Trifluormethyl, Trifluormethoxy, Trifluormethylthio, Trifluorethyl, Trichlorethyl, Difluorchlorethyl, Methoxycarbonyl, Ethoxycarbonyl, Hydroximinomethyl, Hydroximinoethyl, Methoximinomethyl, Ethoximinomethyl, Methoximinoethyl, Ethoximinoethyl oder jeweils unsubstituiertes oder jeweils ein- bis dreifach, gleich oder verschieden durch Fluor, Chlor, Brom, Methyl und/oder Ethyl substituiertes Phenyl, Phenoxy, Benzyl oder Benzyloxy;

$R^2$ für jeweils unsubstituiertes oder jeweils ein-bis fünffach, gleich oder verschieden durch Fluor, Chlor, Brom, Methyl und/oder Ethyl substituiertes Cyclopropyl, Cyclobutyl, Cyclopentyl oder Cyclohexyl, für Dioxolanyl, Dioxanyl oder für einen Rest der Formel

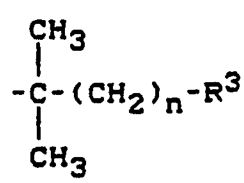

oder für einen Rest der Formel

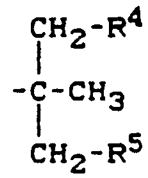

steht und
X für Stickstoff oder eine CH-Gruppe steht,
wobei
$R^3$ für Methoxy, Ethoxy, Methylthio, Ethylthio, Trifluormethoxy, Trifluormethylthio, für Trifluorethoxy, Trifluorethylthio, Trifluorchlorethoxy, für Vinyl, Propenyl, Ethoxycarbonyl, für Cyano, für Dioxolanyl, Dioxanyl oder für jeweils unsubstituiertes oder jeweils ein- bis dreifach, gleich oder verschieden substituiertes Phenyl, Phenoxy, Phenylthio, Benzyloxy oder Benzylthio steht, wobei als Phenylsubstituenten jeweils die bei $R^1$ genannten infrage kommen,
$R^4$ für Wasserstoff, Fluor, Chlor oder Brom steht,
$R^5$ für Wasserstoff, Fluor, Chlor oder Brom steht und
n für eine Zahl 0, 1 oder 2 steht,
ausgenommen die Verbindung 1-Cyclohexyl-3-phenyl-1-(3-pyridyl)-propin-1-ol.

4. Verfahren zur Herstellung von substituierten Pyrimidyl- bzw. Pyridylalkinolen der allgemeinen Formel (I),

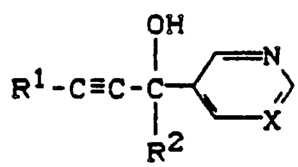
(I)

in welcher
$R^1$ für Alkyl, Alkoxyalkyl, Alkylthioalkyl oder für jeweils unsubstituiertes oder jeweils substituiertes Aryl, Aryloxyalkyl oder Arylthioalkyl steht,
$R^2$ für unsubstituiertes oder substituiertes Cycloalkyl, für Dioxolanyl oder Dioxanyl steht oder für einen der Reste

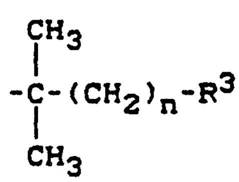 oder 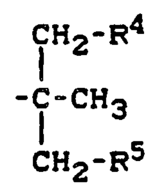 steht und

X für Stickstoff oder eine CH-Gruppe steht,
wobei
$R^3$ für Alkoxy, Alkylthio, Halogenalkoxy, Halogenalkylthio, für Alkenyl, Alkoxycarbonyl oder Cyano, für Dioxolanyl oder Dioxanyl oder für jeweils unsubstituiertes oder substituiertes Aryl, Aryloxy, Arylthio, Arylalkyloxy oder Arylalkylthio steht,
$R^4$ für Wasserstoff oder Halogen steht,
$R^5$ für Wasserstoff oder Halogen steht und
n für eine Zahl 0, 1 oder 2 steht,
ausgenommen die Verbindung 1-Cyclohexyl-3-phenyl-1-(3-pyridyl)-propin-1-ol,
sowie von deren Säureadditionssalzen und Metallsalzkomplexen, dadurch gekennzeichnet, daß man

(a) Pyri(mi)dylketone der Formel (II),

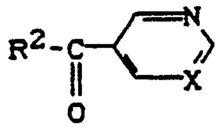 (II)

in welcher
$R^2$ und X die oben angegebene Bedeutung haben,
mit Acetylenverbindungen der Formel (III),

$R^1-C\equiv C-H$ (III)

in welcher
$R^1$ die oben angegebene Bedeutung hat,
gegebenenfalls in Gegenwart eines Verdünnungsmittels und gegebenenfalls in Gegenwart eines Reaktionshilfsmittels umsetzt oder daß man

(b) Acetylenketone der Formel (IV),

$R^1-C\equiv C-\underset{\underset{O}{\|}}{C}-R^2$ (IV)

in welcher
$R^1$ und $R^2$ die oben angegebene Bedeutung haben,
mit metallorganischen Heterocyclen der Formel (V),

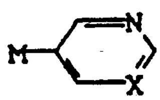 (V)

in welcher
M für Lithium oder für einen Rest -Mg-Hal steht, wobei Hal für Halogen steht und
X die oben angegebene Bedeutung hat,
gegebenenfalls in Gegenwart eines Verdünnungsmittels umsetzt und gegebenenfalls anschliessend eine Säure oder ein Metallsalz addiert.

5. Schädlingsbekämpfungsmittel, gekennzeichnet durch einen Gehalt an mindestens einem substituierten Pyrimidyl- bzw. Pyridylalkinol der Formel (I) nach einem der Ansprüche 1 bis 4.

6. Verwendung von substituierten Pyrimidyl- bzw. Pyridylalkinolen der Formel (I) nach einem der Ansprüche 1 bis 4 zur Bekämpfung von Schädlingen.

7. Verfahren zur Bekämpfung von Schädlingen, dadurch gekennzeichnet, daß man substituierte Pyrimidyl-bzw. Pyridylalkinole der Formel (I) nach einem der Ansprüche 1 bis 4 auf Schädlinge und/oder ihren Lebensraum einwirken läßt.

8. Verfahren zur Herstellung von Schädlingsbekämpfungsmitteln, dadurch gekennzeichnet, daß man substituierte Pyrimidyl- bzw. Pyridylalkinole der Formel (I) nach einem der Ansprüche 1 bis 4 mit Streckmitteln und/oder oberflächenaktiven Mitteln vermischt.

Europäisches Patentamt

# EUROPÄISCHER RECHERCHENBERICHT

Nummer der Anmeldung

EP 89 11 6253

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 272 813 (IMPERIAL CHEMICAL INDUSTRIES)<br>* Ansprüche 1,5,9,11-17 *<br>--- | 1-8 | C 07 D 213/30<br>C 07 D 239/26<br>A 01 N 43/40<br>A 01 N 43/54 |
| A | EP-A-0 209 854 (F. HOFFMANN-LA ROCHE UND CO. AG)<br>* Ansprüche 1,4,12,18,20 *<br>--- | 1-3,5,6 | |
| A | EP-A-0 137 456 (F. HOFFMANN-LA ROCHE UND CO. AG)<br>* Ansprüche 1,3,4,7,8,12,14 *<br>--- | 1-3,5-7 | |
| A | DE-A-2 756 031 (BAYER AG)<br>* Ansprüche 1,3-6 *<br>--- | 1-3,5-8 | |
| A,D | PESTICIDE SCIENCE<br>Band 13, 1982, Seiten 670-678, Oxford, GB; A. ARNOLDI et al.: "Synthesis of Some 3-Phenyl-1-substituted(or 1,1-disubstituted)prop-2-yn-1-ols and Their In-vivo Activity Against Some Phytopathogenic Fungi" * Seite 671, Tabelle 1, Verbindung XIV; Seite 672, Formel (A); Seite 675, Abschnitt 3. *<br>--- | 1-3,6 | **RECHERCHIERTE SACHGEBIETE (Int. Cl.5)** |
| A,D | EP-A-0 221 844 (CIBA-GEIGY AG)<br>* Ansprüche 1,17,22 *<br>--- | 1,5,7 | A 01 N 43/00<br>C 07 D 213/00<br>C 07 D 239/00 |
| A | EP-A-0 144 044 (BAYER AG)<br>* Ansprüche 1-8; Zusammenfassung *; & DE - A - 33 4531 (Cat. D)<br>--- -/- | 4 | |

Der vorliegende Recherchenbericht wurde für alle Patentansprüche erstellt

| Recherchenort | Abschlußdatum der Recherche | Prüfer |
|---|---|---|
| BERLIN | 11-12-1989 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 89 11 6253

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EUROPEAN JOURNAL OF MEDICINAL CHEMISTRY - CHIMICA THERAPEUTICA Band 9, Nr. 6, November/Dezember 1974, Seiten 651-657; M. CUSSAC et al.: "Cétones pyridiniques d'intérêt biologique analogues aux chalcones. VI (*) Alcools dérivés des aza-chalcones doués d'activité dépressive sur le système nerveux central" * Tabelle II * ----- | 1 | |
| | | | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| BERLIN | 11-12-1989 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................
& : Mitglied der gleichen Patentfamilie, übereinstimmendes Dokument

EPO FORM 1503 03.82 (P0403)